# EUROPEAN PATENT APPLICATION

(11) **EP 3 620 537 A2**
(43) Date of publication of application: **11.03.2020**
(21) Application number: 18762895.3
(22) Date of filing: 04.05.2018
(51) Int. Cl.: C12Q 1/6883

(54) **METHOD FOR DIAGNOSIS OF UNSTABLE ATHEROSCLEROTIC PLAQUES**

(30) Priority: 04.05.2017 ES 201730655
(71) Applicant: Universidad del País Vasco/Euskal Herriko Unibertsitatea, 48940 Leioa, Vizcaya (ES); Administración General De La Communidad Autónoma De Euskadi, 01010 Vitoria-Gateiz (Álava) (ES)
(72) Inventor: ALLOZA MORAL, Iraide, 48940 Leioa (Vizcaya) (ES); VANDENBROECK, Koen, 48940 Leioa (Vizcaya) (ES); FREIJO GUERRERO, María del Mar, Vizcaya (ES); VEGA MANRIQUE, Reyes, 48013 Bilbao (Vizcaya) (ES)
(74) Representative: ABG Intellectual Property Law, S.L.
(86) International application number: PCT/ES2018/070338
(87) International publication number: WO 2018/202931

(57) **Abstract**

The present invention relates to a diagnostic method for diagnosing unstable atherosclerotic plaque, a method for determining the probability of a subject suffering from a cerebrovascular disease, and a method for selecting a patient susceptible to being treated with a therapy for removing or stabilizing the carotid atherosclerotic plaque based on determining the expression level of at least one gene selected from the group of genes shown in Table 1. The invention also relates to a kit comprising reagents suitable for determining the expression levels of at least one gene selected from the group of genes shown in Table 1, and optionally reagents for determining the expression levels of one or more housekeeping genes, and uses of said kit.

## Description

### Technical Field

The present invention relates to a diagnostic method for diagnosing unstable atherosclerotic plaque and to a method for determining the probability of a subject suffering from a cerebrovascular disease.

### Background

Cerebrovascular disease is one of the main causes of death and disabilities in developed societies. The risk of suffering from a cerebrovascular accident (CVA) doubles each decade after 55 years of age. Though it may occur at any age, three quarters of CVAs occur in people over 65 years of age. Taking into account the progressive aging of the European population, this disease will be a major health issue in the near future. Specifically, the World Health Organization (WHO) already considers strokes, which are a type of CVA, to be one of the most serious health issues Europe is facing.

Stenosis caused by the formation of atherosclerotic plaque in the carotid artery is one of the main factors for developing a cerebrovascular disease. The plaque can even become unstable and cause a cerebrovascular accident. The process whereby plaque becomes unstable is not a random process and there is evidence that it is a biological process, although the precise mechanism whereby destabilization of the atherosclerotic plaque takes place is unknown. The instability of the atheromatous plaque of the carotid artery is therefore a risk factor for the development of a CVA, even if it is not always associated with the presence of diagnosable symptoms. Atherosclerotic plaques can be defined as stable or asymptomatic when they are made up of a small lipid core and a stable fibrous plaque, or as symptomatic when they are made up of a large lipid core and a thin fibrous plaque with the propensity to become detached. An unstable plaque is related to a higher risk of developing a CVA.

Due to the complexity of the processes that trigger a CVA, the difficulty in finding biomarkers with a real diagnostic value is known in the state of the art (Jickling et al., Stroke 46, 915-920, 2015), and there are no biomarker-based tests that help to identify stable or unstable plaques or to assess the risk of developing a CVA (Hermus et al., Atherosclerosis 213, 21-29, 2010).

There is therefore a need to identify biomarkers with a diagnostic value that allow identifying patients with unstable atherosclerotic plaques and therefore with a higher risk of suffering from a cerebrovascular disease.

### Brief Description of the Invention

In a first aspect, the invention relates to a method for diagnosing unstable atherosclerotic plaques in a subject which comprises:
(i) determining the expression level of at least one gene selected from the group of genes shown in Table 1 or from the group of genes shown in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is diagnosed with unstable atherosclerotic plaque.

In a second aspect, the invention relates to a prognostic method for determining the probability of a subject suffering from a cerebrovascular disease which comprises
(i) determining the expression level of at least one gene selected from the group of genes shown in Table 1 or from the group of genes shown in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject has a high probability of suffering from a cerebrovascular disease.

In a third aspect, the invention relates to a method for selecting a patient susceptible to being treated with a therapy for removing or stabilizing the carotid atherosclerotic plaque which comprises:
(i) determining the expression level of at least one gene selected from the group of genes shown in Table 1 or from the group of genes shown in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is susceptible to being treated with a therapy for removing or stabilizing the carotid atherosclerotic plaque.

In a fourth aspect, the invention relates to a kit comprising reagents suitable for determining the expression levels of at least one gene selected from the group of genes shown in Table 1 or from the group of genes shown in Table 2, and optionally reagents for determining the expression levels of one or more housekeeping genes.

In a fifth aspect, the invention relates to the use of the kit of the invention for diagnosing unstable atherosclerotic plaques in a subject, for determining the probability of a subject suffering from a cerebrovascular disease, or for selecting a patient susceptible to being treated with a therapy for removing or stabilizing the carotid atherosclerotic plaque.

### Brief Description of the Drawings

Figure 1 shows a quality control analysis. (A) Quantification of MYH11-positive cells versus the total number of cells. The data includes the means±SD. *p <0.05. (B) Western blot detection of MYH11 and PECAM1 with actin as a control in SMC cultures in Jurkat and U937 cells.
Figure 2 shows a comparative study between underexpressed and overexpressed genes using the ClusterProfiler method. Comparative analysis based on KEGG.
Figure 3 shows a comparative analysis based on Gene Ontology. GeneRatio corresponds to the number of genes of a specific category. The term enrichment is represented by gray dots (light gray indicates high enrichment and dark gray indicates low enrichment).
Figure 4 shows an MCODE network cluster analysis. It illustrates the cluster identified by the network cluster algorithm, MCODE, showing the interaction between ALK1 signaling, BPM signaling, and TGF-beta, SMAD9, BMP2, ID1, and ID4 signaling. The circles are genes and the rhomboids are protein domains. The light gray lines represent genetic interactions. The light gray circles indicate genes downregulated in S compared to A and the dark gray circles indicate genes upregulated in S compared to A. The dark gray lines represent pathways. The gray lines indicate associations of gene pathways.

### Detailed Description of the Invention

The authors of the present invention have identified different genes that show differential expression in samples obtained from patients diagnosed with symptomatic carotid plaques with respect to patients with asymptomatic plaques (Example 3). Alterations in the expression pattern of at least one of said biomarkers with respect to a reference value are used for diagnosing the stability or instability of an atherosclerotic plaque based on a biological sample from a subject.

### Method for diagnosing unstable atherosclerotic plaques

In a first aspect, the invention relates to a method for diagnosing unstable atherosclerotic plaques in a subject which comprises:
(i) determining the expression level of at least one gene selected from the group of genes shown in Table 1 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is diagnosed with unstable atherosclerotic plaque.

As it is used herein, "diagnostic method" or "diagnose" refers both to the process of trying to determine and/or identify a possible disease in a subject, i.e., the diagnostic process, and to the opinion that is arrived at by means of this process, i.e., the diagnostic opinion. As such, it can also be considered an attempt to classify the state of an individual in different and separate categories that allow making medical decisions concerning the treatment and prognosis. As those skilled in the art will understand, the diagnosis of unstable atherosclerotic plaque, is not necessarily, although it is preferably, correct for 100% of the subjects to be diagnosed or evaluated. However, the term requires enabling identification of a statistically significant part of the subjects who have an unstable atherosclerotic plaque. One skilled in the art can readily determine if a subject is statistically significant by using various well-known statistical evaluation tools, for example, the determination of confidence intervals, the determination of the p-value, Student's t-test, Mann-Whitney test, etc. Details can be found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are preferably 0.05, 0.01, 0.005, or lower.

As it is used herein, the term "atherosclerosis" refers to a pathology characterized by the deposition and infiltration of lipid substances in the walls of thick medium-sized arteries. The cells of the arterial wall interpret this deposition as an invasion and activate circulating monocytes of the immune system, which penetrate the arterial wall, turn into macrophages and begin to phagocyte LDL particles, generating an inflammatory process. The inflammation in turn causes the multiplication and migration of the smooth muscle cells of the wall, which causes a gradual stricture of the diameter of the artery. The specific thickening constitutes an atheromatous plaque. Included among those diseases presenting with atherosclerosis characterized by the stricture of the arteries due to atheromatous plaques, and accordingly obstruction of the blood flow or ischemia, depending on the artery of the organ involved, are: ischemic heart disease (with acute myocardial infarction of the heart being the maximum representative), cerebrovascular disease (in the form of apoplexy or cerebral thrombosis or intracerebral hemorrhage, in the central nervous system), intermittent claudication (with the most serious form being arterial ischemia of the lower extremities), erectile dysfunction, ischemic colitis (an area of inflammation, irritation, and swelling, caused by interference with the blood flow in the colon, in the arteries of the intestine), and aortic aneurism.

As it is used herein, "atherosclerotic plaque," also known in the art as "atheromatous plaque," refers to the buildup of lipid substances on arterial walls according to the definition of "atherosclerosis" until forming a plaque formed by lipids, macrophages, and smooth muscle cells. The atherosclerotic plaque can be stable or unstable.

The term "stable plaque," also known in the art as "stable atherosclerotic plaque" or "asymptomatic plaque," has been used to describe atherosclerotic plaques that are not particularly susceptible to rupture. They are generally characterized by having a thick fibrous cap over a small lipid core.

The term "unstable atherosclerotic plaque," also known in the art as "unstable plaque" or "symptomatic plaque," has been used to describe atherosclerotic plaques that are particularly susceptible to rupture. They are generally characterized as having an inflamed thin fibrous cap over a very large lipid core. It consists of a mixture of white blood cells (primarily macrophages), smooth muscle cells (SMCs), and lipids (including cholesterol) in the wall of an artery. They are particularly unstable and have the propensity to cause sudden problems, such as a heart attack or apoplexy. The characteristics that define an unstable plaque include: inflammatory activity, a thin fibrous cap, a large lipid core, visible intra-plaque ulceration, bleeding, and rupture.

In a particular embodiment of the methods of the invention, the unstable atherosclerotic plaque is a carotid plaque. The blood supply to the carotid artery begins in the aortic arch; the carotid artery is divided into the internal carotid artery (which supplies blood to the brain) and the external carotid artery. The atherosclerotic plaque usually builds up in that division of the carotid artery, which causes stricture, also known as stenosis.

The term "subject" or "individual" or "patient" refers to any subject, particularly a mammalian subject, for whom the diagnosis, prognosis, or treatment is desired. Mammalian subjects include human beings, domestic animals, farm animals, and zoo animals, animals used for sports, or domestic animals such as dogs, cats, guinea pigs, rabbits, rats, mice, horses, cattle, cows, and so on and so forth. In a preferred embodiment of the invention, the subject is a mammal. In a more preferred embodiment of the invention, the subject is a human being. In another particular embodiment, the subject is a subject who potentially has a disease associated with an alteration in atherosclerotic plaques.

In a particular embodiment, the subject whose diagnosis is to be determined according to the first method of the invention has already had a cerebrovascular complication prior to being diagnosed with unstable atherosclerotic plaque. In a particular embodiment, said cerebrovascular complication is selected from the group consisting of a transient ischemic attack (TIA), apoplexy, and amaurosis fugax.

According to the methods of the invention, the expression level is determined in a biological sample from the subject.

As it is used herein, "biological sample" refers to a biological material isolated from a subject. The biological sample contains any material suitable for detecting the expression level of a gene and can be a material comprising genetic material from the subject. The biological sample may comprise cellular and/or non-cellular material from the subject, preferably non-cellular material. In a particular embodiment, the sample comprises genetic material, for example, DNA, genomic DNA (gDNA), complementary DNA (cDNA), RNA, mRNA, etc., from the subject under study. In a particular embodiment, the genetic material is RNA. The sample can be isolated from any biological tissue or fluid, such as, for example, blood, saliva, plasma, serum, urine, cerebrospinal fluid (CSF), feces, nasal, buccal, or buccopharyngeal swabs, a specimen, a specimen obtained from a biopsy, and a paraffin-embedded tissue sample. The methods for isolating samples are well known to those skilled in the art.

Before analyzing the sample, it will often be desirable to perform one or more operations for preparing said sample to separate the molecule to be determined from other molecules found in the sample. In a particular embodiment, the molecules are nucleic acids, DNA, and/or RNA. These operations for preparing the samples include manipulations such as: concentration, suspension, extraction of intracellular material (for example, nucleic acids of whole tissue/cell samples and the like), nucleic acid amplification, fragmentation, transcription, labeling, and/or extension reactions. These methods are well known to one skilled in the art. There are also commercial kits available for mRNA purification including, without limitation, miRNeasy Mini kit from Qiagen, miRNA isolation kits from Life Technologies, the mirPremier microRNA isolation kit from Sigma-Aldrich, and the High Pure miRNA isolation kit from Roche. In a particular embodiment, RNA integrity was analyzed using RNA 6000 Nano Chips in the Agilent 2100 bioanalyzer (Agilent Technologies, Palo Alto, CA, USA).

In a particular embodiment, the biological sample is a sample containing cells from an atherosclerotic plaque, more particularly a carotid atherosclerotic plaque. One skilled in the art knows the techniques for detecting atherosclerotic plaques, such as calculating the ankle-brachial index, magnetic resonance arteriogram, duplex ultrasound, and the hemodynamics for localizing purposes, or contrast arteriogram.

The atheroma plaque can be obtained by means of carotid endarterectomy, for example. Once the plaque is obtained, the cells from an atherosclerotic plaque can be isolated using various known techniques, such as cell explants, enzymatic digestion combined with culturing in media specific for each cell type and/or by means of separation techniques. By way of non-limiting illustrative example, the separation can be done by means of isopycnic sedimentation, centrifugal elutriation, immunomagnetic separation, such as the use of polystyrene beads with paramagnetic properties (Dynabeads©), superparamagnetic particles (MACS© microbeads); flow cytometry, fluorescence-activated cell sorter (FACS).

In a particular embodiment, the cells extracted from the atherosclerotic plaque are endothelial cells. In another particular embodiment, the cells extracted from the atherosclerotic plaque are macrophages. In a more preferred embodiment, the cells extracted from the atherosclerotic plaque are smooth muscle cells (SMCs).

The smooth muscle cells from an atherosclerotic plaque can be obtained by any method known to one skilled in the art. In a particular embodiment, recently obtained carotid artery tissue samples subsequently digested with enzymes that allow separating the different cell types, such as collagenase type I by way of non-limiting illustrative example, are processed. They are then cultured in dishes with media specific for culturing the cells of interest, specifically smooth muscle cells, such as M231 by way of non-limiting illustrative example.

In a particular embodiment, the SMCs are positive for the specific marker MYH11 and/or negative for the specific marker PECAM1. In a more particular embodiment, the SMCs are MYH11-positive and PECAM1-negative.

As it is used herein, "MYH11" refers to the protein Myosin-11, which in humans has the sequence shown in the Uniprot database under accession number P35749 (date 15/03/2017).

"PECAM1" or CD31 refers to the plate-endothelial cell adhesion molecule. The sequence in humans corresponds to that shown in the Uniprot database under accession number P16284 (date 12/04/2017).

Various techniques for determining if a cell is positive or negative for a specific marker are known.

The presence/absence of a marker in a cell can be determined, for example, by means of flow cytometry using conventional methods and apparatus. For example, a BD LSR II flow cytometer (BD Biosciences Corp., Franklin Lakes, NJ, USA) with commercially available antibodies and following protocols known in the art can be used. Therefore, cells emitting a signal for a specific cell surface marker that is stronger than the background noise can be selected. The background signal is defined as the signal strength produced by a non-specific antibody of the same isotype as the specific antibody used for detecting each surface marker in the conventional FACS analysis. To consider a marker positive, the specific signal observed must be 20%, preferably, 30%, 40%, 50%, 60%, 70%, 80%, 90%, 500%, 1000%, 5000%, 10000%, or higher, stronger than the background signal using conventional methods and apparatus (for example using a FACSCalibur flow cytometer (BD Biosciences Corp., Franklin Lakes, NJ, USA) and commercially available antibodies). Otherwise, the cell is considered negative for said marker.

In a particular embodiment, the determination of the expression levels of the genes is carried out using a sample derived from an entire atherosclerotic plaque and it therefore contains genetic material derived from all the cell types of said plaque.

In a first step, the diagnostic method of the invention comprises determining the expression level of at least one gene selected from the group of genes shown in Table 1 or from the group of genes shown in Table 2 in a biological sample from said subject.

**Table 1. List of genes which show differential gene expression according to the described method, with the corresponding category indicated in the description. The Gene Identifier refers to the name in the Ensembl database.**

| No. | Gene Identifier | Symbol | GROUP |
|---|---|---|---|
| 1 | ENSG00000165507 | C10orf10 | 3 |
| 2 | ENSG00000152049 | KCNE4 | 1 |
| 3 | ENSG00000117069 | ST6GALNAC5 | 3 |
| 4 | ENSG00000135424 | ITGA7 | 1,2,5,6 |
| 5 | ENSG00000128285 | MCHR1 | 6 |
| 6 | ENSG00000174564 | IL20RB | 4 |
| 7 | ENSG00000163815 | CLEC3B | 1,2 |
| 8 | ENSG00000075643 | MOCOS | 7 |
| 9 | ENSG00000074410 | CA12 | 2 |
| 10 | ENSG00000174807 | CD248 | 5 |
| 11 | ENSG00000133454 | MYO18B | 1 |
| 12 | ENSG00000179542 | SLITRK4 | 4 |
| 13 | ENSG00000161570 | CCL5 | 4 |
| 14 | ENSG00000148948 | LRRC4C | 4 |
| 15 | ENSG00000117289 | TXNIP | 1 |
| 16 | ENSG00000123689 | G0S2 | 3 |
| 17 | ENSG00000162706 | CADM3 | 6 |
| 18 | ENSG00000120088 | CRHR1 | 1,3 |
| 19 | ENSG00000184557 | SOCS3 | 1,2,4 |
| 20 | ENSG00000176438 | SYNE3 | 1 |
| 21 | ENSG00000161958 | FGF11 | 6 |
| 22 | ENSG00000234745 | HLA-B | 3,4,6 |
| 23 | ENSG00000164236 | ANKRD33B | 7 |
| 24 | ENSG00000170624 | SGCD | 1 |
| 25 | ENSG00000259649 | RP11-351M8.1 | 4 |
| 26 | ENSG00000172201 | ID4 | 1,2,3,4 |
| 27 | ENSG00000119938 | PPP1R3C | 6 |
| 28 | ENSG00000114268 | PFKFB4 | 4 |
| 29 | ENSG00000110076 | NRXN2 | 6 |
| 30 | ENSG00000136378 | ADAMTS7 | 1,2,4 |
| 31 | ENSG00000152256 | PDK1 | 6 |
| 32 | ENSG00000112837 | TBX18 | 1,4,6 |
| 33 | ENSG00000162733 | DDR2 | 1,2,4,5 |
| 34 | ENSG00000179348 | GATA2 | 1,2,4,5 |
| 35 | ENSG00000120693 | SMAD9 | 1,4 |
| 36 | ENSG00000164736 | SOX17 | 1,3,4,5 |
| 37 | ENSG00000035664 | DAPK2 | 5,6 |
| 38 | ENSG00000144730 | IL17RD | 4 |
| 39 | ENSG00000133216 | EPHB2 | 1,4,6 |
| 40 | ENSG00000166960 | CCDC178 | 7 |
| 41 | ENSG00000120279 | MYCT1 | 4 |
| 42 | ENSG00000121039 | RDH10 | 1 |
| 43 | ENSG00000183578 | TNFAIP8L3 | 2 |
| 44 | ENSG00000145681 | HAPLN1 | 3 |
| 45 | ENSG00000158125 | XDH | 1,3,4,6 |
| 46 | ENSG00000139174 | PRICKLE1 | 1,4 |
| 47 | ENSG00000182368 | FAM27A | 4 |
| 48 | ENSG00000118137 | APOA1 | 3,4,5,6 |
| 49 | ENSG00000169684 | CHRNA5 | 3 |
| 50 | ENSG00000167644 | C19orf33 | 4 |
| 51 | ENSG00000116106 | EPHA4 | 1,4,5,6 |
| 52 | ENSG00000125968 | ID1 | 1,2,3,4,5,6 |
| 53 | ENSG00000123572 | NRK | 1 |
| 54 | ENSG00000121361 | KCNJ8 | 1,3,6 |
| 55 | ENSG00000091129 | NRCAM | 1,3,4,5 |
| 56 | ENSG00000175746 | C15orf54 | 7 |
| 57 | ENSG00000172602 | RND1 | 4,6 |
| 58 | ENSG00000095752 | IL11 | 2,6 |
| 59 | ENSG00000175866 | BAIAP2 | 2,4,6 |
| 60 | ENSG00000127329 | PTPRB | 1 |
| 61 | ENSG00000144366 | GULP1 | 4 |
| 62 | ENSG00000143140 | GJA5 | 1 |
| 63 | ENSG00000176399 | DMRTA1 | 6 |
| 64 | ENSG00000168497 | SDPR | 1 |
| 65 | ENSG00000010319 | SEMA3G | 1,4,5,6 |
| 66 | ENSG00000254656 | RTL1 | 7 |
| 67 | ENSG00000178445 | GLDC | 6 |
| 68 | ENSG00000162496 | DHRS3 | 1,4 |
| 69 | ENSG00000102554 | KLF5 | 1,4 |
| 70 | ENSG00000172159 | FRMD3 | 3 |
| 71 | ENSG00000164283 | ESM1 | 1,6 |
| 72 | ENSG00000235109 | ZSCAN31 | 4 |
| 73 | ENSG00000132321 | IQCA1 | 7 |
| 74 | ENSG00000008394 | MGST1 | 2,6 |
| 75 | ENSG00000164530 | PI16 | 1,4 |
| 76 | ENSG00000150722 | PPP1R1C | 2 |
| 77 | ENSG00000116741 | RGS2 | 3,4 |
| 78 | ENSG00000180660 | MAB21L1 | 7 |
| 79 | ENSG00000155011 | DKK2 | 4 |
| 80 | ENSG00000126950 | TMEM35 | 7 |
| 81 | ENSG00000179104 | TMTC2 | 2 |
| 82 | ENSG00000118777 | ABCG2 | 3, 6 |
| 83 | ENSG00000125848 | FLRT3 | 1,4,5,6 |
| 84 | ENSG00000108375 | RNF43 | 2,3,4,6 |
| 85 | ENSG00000125845 | BMP2 | 1,2,3,4,5,6 |
| 86 | ENSG00000138759 | FRAS1 | 1 |
| 87 | ENSG00000108556 | CHRNE | 6 |
| 88 | ENSG00000163273 | NPPC | 1,2,4,6 |
| 89 | ENSG00000162595 | DIRAS3 | 4 |
| 90 | ENSG00000109511 | ANXA10 | 4 |
| 91 | ENSG00000154645 | CHODL | 2,4 |
| 92 | ENSG00000138772 | ANXA3 | 1,3,5 |
| 93 | ENSG00000087494 | PTHLH | 1,2,3,4,6 |

**Table 2. List of genes which show differential gene expression according to the described method. The Gene Identifier refers to the name in the Ensembl database. Genes with a differential gene expression FC value <-1.2 and >1.2 with significance p≤0.05.**

| No. | Gene Identifier | Symbol |
|---|---|---|
| 1 | ENSG00000184014 | DENND5A |
| 2 | ENSG00000101577 | LPIN2 |
| 3 | ENSG00000165406 | MARCH8 |
| 4 | ENSG00000122861 | PLAU |
| 5 | ENSG00000073060 | SCARB1 |
| 6 | ENSG00000059804 | SLC2A3 |
| 7 | ENSG00000132563 | REEP2 |
| 8 | ENSG00000165948 | IFI27L1 |
| 9 | ENSG00000156103 | MMP16 |
| 10 | ENSG00000116711 | PLA2G4A |
| 11 | ENSG00000155850 | SLC26A2 |

**Table 3. List of genes of Table 1 which show differential gene expression according to the described method and which have been validated in a different patient cohort, with the corresponding category indicated in the description. Genes identified in a validation analysis, performed in a cohort of 190 carotid artery atheroma plaque samples, of those genes previously identified in the transcriptomic study performed in 14 atheroma plaque SMC samples. The Gene Identifier refers to the name in the Ensembl database.**

| No. | Gene Identifier | Symbol | GROUP |
|---|---|---|---|
| 3 | ENSG00000117069 | ST6GALNAC5 | 3 |
| 4 | ENSG00000135424 | ITGA7 | 1,2,5,6 |
| 8 | ENSG00000075643 | MOCOS | 7 |
| 9 | ENSG00000074410 | CA12 | 2 |
| 20 | ENSG00000176438 | SYNE3 | 1 |
| 28 | ENSG00000114268 | PFKFB4 | 4 |
| 30 | ENSG00000136378 | ADAMTS7 | 1,2,4 |
| 31 | ENSG00000152256 | PDK1 | 6 |
| 32 | ENSG00000112837 | TBX18 | 1,4,6 |
| 33 | ENSG00000162733 | DDR2 | 1,2,4,5 |
| 36 | ENSG00000164736 | SOX17 | 1,3,4,5 |
| 38 | ENSG00000144730 | IL17RD | 4 |
| 48 | ENSG00000118137 | APOA1 | 3,4,5,6 |
| 52 | ENSG00000125968 | ID1 | 1,2,3,4,5,6 |
| 53 | ENSG00000123572 | NRK | 1 |
| 60 | ENSG00000127329 | PTPRB | 1 |
| 68 | ENSG00000162496 | DHRS3 | 1,4 |
| 69 | ENSG00000102554 | KLF5 | 1,4 |
| 70 | ENSG00000172159 | FRMD3 | 3 |
| 75 | ENSG00000164530 | PI16 | 1,4 |
| 82 | ENSG00000118777 | ABCG2 | 3,6 |
| 86 | ENSG00000138759 | FRAS1 | 1 |

As it is herein used, the term "expression level" of a gene refers to the measurable amount of gene product in a sample from the subject, wherein the gene product can be a product of transcription or a product of translation of said gene. Accordingly, the expression level may correspond to a nucleic acid of the gene (such as mRNA or cDNA) or a polypeptide encoded by said gene. The expression level is derived from the sample from a subject and/or from a reference sample or samples, and it may be detected *de novo* or it may correspond to a previous determination.

It is possible to determine the expression levels of the markers by means of determining the expression levels of the proteins encoded by the genes because if the expression of the genes increases, an increase in the amount of the corresponding protein can be expected to take place, and if the expression of the genes decreases, a decrease in the amount of the corresponding protein can be expected to take place.

Therefore, in a particular embodiment the expression level of a gene can be determined by means of quantifying the level of protein expressed by said gene.

The level of a protein can be quantified by means of any conventional method that allows detecting and quantifying said protein in a sample from a subject. By way of non-limiting illustration, the levels of said protein can be quantified, for example, by means of using antibodies that have the ability to bind to the protein of interest (or to fragments thereof which contain an antigenic determinant) and the subsequent quantification of the complexes that are formed. The antibodies that are used in these assays can be labeled or unlabeled. Non-limiting illustrative examples of markers that can be used include radioactive isotopes, enzymes, fluorophores, chemiluminescent reagents, enzymatic substrates or cofactors, enzyme inhibitors, particles, dyes, etc. There is a wide range of known assays that can be used in the present invention which use unlabeled antibodies (primary antibody) and labeled antibodies (secondary antibody); among others, these techniques include by way of non-limiting illustration, Western blot, ELISA (enzyme-linked immunosorbent assay), RIA (radioimmunoassay), competitive EIA (competitive enzyme immunoassay), DAS-ELISA (double antibody sandwich ELISA), immunocytochemical techniques, and immunohistochemical techniques, techniques based on the use of protein biochips or microarrays that include specific antibodies or assays based on colloidal precipitation in formats such as dipsticks. Other ways for detecting and quantifying a protein include affinity chromatography techniques, ligand binding assays, etc. When an immunological method is used, any antibody or reagent that is known to bind to the protein of interest with a high affinity can be used for detecting the amount of said protein. However, it is preferable to use an antibody, for example, polyclonal sera, hybridoma supernatants, or monoclonal antibodies, antibody fragments, Fv, Fab, Fab', and F(ab')2, scFv, diabodies, triabodies, tetrabodies, and humanized antibodies.

In a particular embodiment, quantification of the protein levels is performed by means of Western blot, ELISA, or by means of a protein array.

In another particular embodiment, quantification of the expression level of a gene is carried out based on the mRNA encoded by said gene, or alternatively based on the complementary DNA (cDNA) of said mRNA. Therefore, in a particular embodiment quantification of the expression level of a gene comprises quantification of the mRNA containing the sequence encoded by said gene, or a fragment of said mRNA, or quantification of the cDNA of said mRNA, or a fragment of said cDNA, or mixtures thereof.

To that end, the biological sample can be treated for physically or mechanically breaking up the structure of the tissue or cell, releasing the intracellular components into an aqueous or organic solution for preparing the nucleic acids. The nucleic acids are extracted by means of methods known to one skilled in the art and are commercially available (Sambroock, J., et al., "Molecular cloning: a Laboratory Manual," 3rd ed., Cold Spring Harbor Laboratory Press, N.Y., Vol. 1-3.)

Virtually any conventional method can be used within the framework of the present invention for detecting and quantifying the level of a gene. By way of non-limiting illustration, the level of mRNA encoded by said gene can be quantified by means of using conventional methods, for example, methods comprising amplification of mRNA and quantification of the product of amplification of said mRNA, such as electrophoresis and staining, or alternatively by means of Southern blot and the use of suitable probes, Northern blot and the use of specific probes of the mRNA encoded by a gene or of its corresponding cDNA, nuclease S1 mapping, RT-LCR, hybridization, microarrays, etc., preferably by means of real-time quantitative PCR using a suitable label. Similarly, the levels of the cDNA corresponding to said mRNA encoded by the gene can also be quantified by means of using conventional techniques; in this case, the method of the invention includes a step of synthesizing the corresponding cDNA by means of reverse transcription (RT) of the corresponding mRNA followed by amplification and quantification of the product of amplification of said cDNA. Conventional methods for quantifying expression levels can be found, for example, in Sambrook et *al.,* 2001 (mentioned above).

To normalize the expression values of mRNA among the different samples, it is possible to compare the expression levels of the mRNA of interest in the test samples with the expression of a control RNA. As it used herein, a "control RNA" refers to an RNA the expression levels of which do not change or change only by limited amounts. Preferably, the control RNA is an mRNA derived from housekeeping genes and encodes proteins that are constitutively expressed and carry out essential cell functions. Preferred housekeeping genes for use in the present invention include 18S ribosomal protein, β-2-microglobulin, ubiquitin, cyclophilin, GAPDH, PSMB4, tubulin, and β-actin.

The determination of levels of RNA, particularly levels of mRNA, can be carried out by any method known in the state of the art, such as qPCR, Northern blot, RNA dot blot, TaqMan, methods based on the serial analysis of gene expression (SAGE) including variants such as LongSAGE and SuperSAGE, microarrays. The determination of levels of mRNA can also be carried out by fluorescence *in situ* hybridization (FISH). The detection can be carried out in individual samples or in tissue microarrays. In a particular embodiment, the expression levels of RNA in a sample from a subject to be diagnosed with unstable atherosclerotic plaque are determined by real-time PCR (RT-PCR).

Real-time PCR (also known as quantitative PCR, real-time quantitative PCR, or RTQ-PCR) is a method for the simultaneous quantification and amplification of DNA (Expert Rev. Mol, Diagn. 2005 (2): 209-19). The DNA is amplified specifically by means of polymerase chain reaction. After each round of amplification, the DNA is quantified. Common quantification methods include the use of fluorescent dyes intercalated with double-stranded DNA and modified DNA oligonucleotides (called probes) which are fluorescent when they hybridize with DNA.

In another particular embodiment, expression levels are determined by means of RNA sequencing or RNAseq.

In a particular embodiment, quantification of the expression levels of a gene is performed by means of a quantitative polymerase chain reaction (PCR) or a DNA or RNA array or by means of nucleotide hybridization techniques based on mRNA or its corresponding cDNA.

As it is used herein, RNA-seq refers to a deep sequencing technology providing a more precise level of measurement of the transcriptions. This technology involves making a collection of cDNA fragments that are flanked by specific constant sequences (known as adaptors) required for sequencing. Adaptors contain several different functional elements required for sequencing and may contain one or more optional elements. Generally, a population of RNA (total or fractionated, such as poly (A) +) is converted into a library of cDNA fragments with adaptors bound at one or both ends. Each molecule, with or without amplification, is then subjected to high throughput sequencing to obtain short sequences of one end (single-end sequencing) or both ends (paired-end sequencing). The reads are conventionally of 30-400 bp, depending on the DNA sequencing technology used. Any high-throughput sequencing technology can be used for RNA-Seq, for example the HiSeq2500 system (Illumina) and Roche 454 Life Science system. After sequencing, the resulting reads are aligned with a reference genome or reference transcripts, or are assembled *de novo* without the genomic sequence to produce a genome scale transcriptional map consisting of the transcriptional structure and/or expression level of each gene. In a preferred embodiment, quantification is performed using the Hiseq2500 instrument using 2 µg of RNA from a total RNA library.

Once the values of the expression level have been obtained, a fold change (FC) value which can be obtained by means of the DeSeq2 method, and the adjusted p-value (P_{adjust}) which can be calculated using the false discovery rate (FDR) method, can be determined.

In a particular embodiment, the method comprises determining the expression level of at least 1 gene, at least 2 genes, at least 3 genes, at least 4 genes, at least 5 genes, at least 6 genes, at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes, at least 20 genes, at least 21 genes, at least 22 genes, at least 23 genes, at least 24 genes, at least 25 genes, at least 26 genes, at least 27 genes, at least 28 genes, at least 29 genes, at least 30 genes, at least 31 genes, at least 32 genes, at least 33 genes, at least 34 genes, at least 35 genes, at least 36 genes, at least 37 genes, at least 38 genes, at least 39 genes, at least 40 genes, at least 41 genes, at least 42 genes, at least 43 genes, at least 44 genes, at least 45 genes, at least 46 genes, at least 47 genes, at least 48 genes, at least 49 genes, at least 50 genes, at least 51 genes, at least 52 genes, at least 53 genes, at least 54 genes, at least 55 genes, at least 56 genes, at least 57 genes, at least 58 genes, at least 59 genes, at least 60 genes, at least 61 genes, at least 62 genes, at least 63 genes, at least 64 genes, at least 65 genes, at least 66 genes, at least 67 genes, at least 68 genes, at least 69 genes, at least 70 genes, at least 71 genes, at least 72 genes, at least 73 genes, at least 74 genes, at least 75 genes, at least 76 genes, at least 77 genes, at least 78 genes, at least 79 genes, at least 80 genes, at least 81 genes, at least 82 genes, at least 83 genes, at least 84 genes, at least 85 genes, at least 86 genes, at least 87 genes, at least 88 genes, at least 89 genes, at least 90 genes, at least 91 genes, at least 92 genes, or of the 93 genes included in Table 1.

The present invention refers to any combination of genes 1-93 from Table 1.

In a particular embodiment, the method for diagnosing unstable atherosclerotic plaques comprises determining the expression level of all the genes from Table 1.

In a particular embodiment, the method comprises determining the expression level of at least 1 gene, at least 2 genes, at least 3 genes, at least 4 genes, at least 5 genes, at least 6 genes, at least 7 genes, at least 8 genes, at least 9 genes, at least 10 genes or of those included in Table 2.

In another particular embodiment, the method for diagnosing unstable atherosclerotic plaques comprises determining the expression level of at least one gene from group 1, at least one gene from group 2, at least one gene from group 3, at least one gene from group 4, at least one gene from group 5, and/or at least one gene from group 6.

In group 1 are those genes involved in circulatory system development, vascular system development, or tissue development. Specifically, they are genes ADAMTS7, ANXA3, BMP2, CLEC3B, CRHR1, DDR2, DHRS3, EPHA4, EPHB2, ESM1, FLRT3, FRAS1 GATA2, ID1, ID4, ITGA7, KCNJ8, KLF5, MYO18B, NPPC, NRCAM, NRK, PI16, PRICKLE1, PTHLH, PTPRB, RDH10, SEMA3G, SGCD, SMAD9, SOCS3, SOX17, TBX18, XDH, GJA5, TXNIP, SYNE3, KCNE4, and SDPR.

In group 2 are those genes involved in bone mineralization and regulation of osteoclast, osteoblast, and chondrocyte differentiation. Specifically, they are genes ADAMTS7, BAIAP2, BMP2, CA12, CLEC3B, CHODL, DDR2, GATA2, ID1, ID4, IL11, ITGA7, MGST1, NPPC, PPP1R1C, PTHLH, RNF43, SOCS3, TMTC2, and TNFAIP8L3.

In group 3 are those genes involved in pathological processes. Specifically, they are genes ABCG2, ANXA3, APOA1, BMP2, C10orf10, CHRNA5, CRHR1, G0S2, HAPLN1, HLA-B, ID1, ID4, KCNJ8, NRCAM, PTHLH, RGS2, RNF43, SOX17, ST6GALNAC5, XDH, and FRMD3.

In group 4 are those genes involved in cell differentiation, signal transduction, adipocyte differentiation, and survival. Specifically, they are genes ADAMTS7, APOA1, BAIAP2, BMP2, CHODL, DDR2, DHRS3, DKK2, EPHA4, EPHB2, FLRT3, GATA2, HLA-B, ID1, ID4, IL17RD, KLF5, LRRC4C, NPPC, NRCAM, PI16, PRICKLE1, PTHLH, RGS2, RND1, RNF43, SEMA3G, SLITRK4, SMAD9, SOCS3, SOX17, TBX18, XDH, FAM27A, RP11-351M8.1, ZSCAN31, ANXA10, C19orf33, CCL5, DIRAS3, GULP1, IL20RB, MYCT1, and PFKFB4.

In group 5 are those genes involved in cell migration. Specifically, they are genes BMP2, NRCAM, CD248, ANXA3, DAPK2, DDR2, GATA2, SOX17, ID1, APOA1, FLRT3, SEMA3G, ITGA7, and EPHA4.

In group 6 are those genes involved in protein binding. Specifically, they are genes ABCG2, APOA1, BAIAP2, BMP2, CADM3, DAPK2, DMRTA1, EPHA4, EPHB2, ESM1, FGF11, FLRT3, GLDC, HLA-B, ID1, IL11, ITGA7, KCNJ8, MCHR1, MGST1, NPPC, NRXN2, PDK1, PTHLH, RND1, RNF43, SEMA3G, TBX18, XDH, PPP1R3C, and CHRNE.

In another preferred embodiment, the method for diagnosing unstable atherosclerotic plaques according to the invention comprises determining the expression level of all the genes from group 1.

In another particular embodiment, the method for diagnosing unstable atherosclerotic plaques according to the invention comprises determining the expression level of all the genes from group 2.

In another particular embodiment, the method for diagnosing unstable atherosclerotic plaques according to the invention comprises determining the expression level of all the genes from group 3.

In another particular embodiment, the method for diagnosing unstable atherosclerotic plaques according to the invention comprises determining the expression level of all the genes from group 4.

In another particular embodiment, the method for diagnosing unstable atherosclerotic plaques according to the invention comprises determining the expression level of all the genes from group 5.

In another particular embodiment, the method for diagnosing unstable atherosclerotic plaques according to the invention comprises determining the expression level of all the genes from group 6.

In another particular embodiment, the method for diagnosing unstable atherosclerotic plaques according to the invention comprises determining the expression level of all the genes from Table 3.

In another particular embodiment, the method for diagnosing unstable atherosclerotic plaques according to the invention comprises determining the expression level of all the genes from group 1, all the genes from group 2, all the genes from group 3, all the genes from group 4, all the genes from group 5, and all the genes from group 6.

In a second step, the method for diagnosing atherosclerotic plaques of the invention comprises comparing the expression level obtained in step (i) with a corresponding reference value.

The "reference value" or "reference level" according to any of the methods of the invention can be an absolute value, a relative value, a value having an upper and/or lower limit, a range of values, an average value, a value of the median, a mean value, or a value compared with a particular control, or a starting value. A reference value can be based on an individual sample value, such as a value obtained for a sample from the subject being tested, for example. The reference value can be based on a large number of samples, for example on a population of subjects of the same age, or it can be based on a set of samples including or excluding the sample to be tested. Several considerations are taken into account when the reference value of a gene is determined. Among said considerations are age, weight, sex, general physical condition of the patient, and the like. For example, equal amounts of a group of at least 2, at least 10, at least 100 to preferably more than 1000 subjects, preferably classified according to the preceding considerations, for example according to several categories of age, are taken as the reference group.

In a particular embodiment, the reference value of a gene corresponds to the expression level of said gene in a healthy subject. In another particular embodiment, the reference value of a gene corresponds to the expression level of said gene in a subject who does not have unstable atherosclerotic plaques. In another particular embodiment, the reference value of a gene corresponds to the expression level of said gene in an atherosclerotic plaque cell sample from a subject who has stable atherosclerotic plaques.

Once the reference value is established for the corresponding gene of interest, the expression level of the gene of interest determined in a biological sample from the subject is compared with the reference value, and therefore can be assigned as an "increase" or "decrease" in the expression value of said gene and can enable determining if the subject has unstable atherosclerotic plaques.

As it is used herein in relation to the expression level of a gene, the term "increase in the expression level", refers to the expression level being at least 5%, at least 10%, at least 20%, at least one 30%, at least one 40%, at least 50%, at least 60%, at least 70%, at least 80%, at least 90%, or at least 100% higher compared with the corresponding reference value.

As it is used herein in relation to the expression level of a gene, the term "decrease in the expression level" refers to the expression level decreasing at least 5%, at least 10%, at least 20%, at least one 30%, at least one 40%, at least 50%, at least 60%, at least 70%, at least 80%, at least 90%, or at least 100% compared with the corresponding reference value.

According to the diagnostic method for diagnosing atherosclerotic plaques in a subject, if the at least one gene determined in step (i) is one of genes 1-33 from Table 1 and its expression is increased with respect to the reference value, and/or wherein if the at least one gene determined in step (i) is one of genes 34-93 from Table 1 and its expression is reduced with respect to the reference value, then the subject is diagnosed with unstable atherosclerotic plaque.

Alternatively, according to the diagnostic method for diagnosing atherosclerotic plaques in a subject, if the at least one gene determined in step (i) is one of genes 1-6 from Table 2 and its expression is increased with respect to the reference value, and/or wherein if the at least one gene determined in step (i) is one of genes 6-11 from Table 2 and its expression is reduced with respect to the reference value, then the subject is diagnosed with unstable atherosclerotic plaque.

### Method for determining the probability of a subject suffering from a cerebrovascular disease

The presence of an unstable atherosclerotic plaque is associated with an increase in the risk of suffering from a cerebrovascular disease (Insull, W. Am. J. Med. 122 (1 suppl), S3-S14 (2009). Accordingly, changes in the expression levels of those genes that are markers of the presence of an unstable atherosclerotic plaque also allow classifying subjects based on their risk of suffering from a cerebrovascular disease.

Therefore, in a second aspect the invention relates to a prognostic method for determining the probability of a subject suffering from a cerebrovascular disease which comprises
(i) determining the expression level of at least one gene selected from the group of genes shown in Table 1 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject has a high probability of suffering from a cerebrovascular disease.

As it is used herein in the context of predicting methods of the invention, the term "determining the probability" or "predicting" refers to the prediction of a subject having a cerebrovascular disease, or it refers to the prediction of the progression of the cerebrovascular disease in a subject. As one skilled in the art will understand, said determination is not usually correct for all (i.e., 100%) of the patients to be identified. However, the term requires being able to identify a significant part of the subjects. One skilled in the art can readily determine if a part is statistically significant using several well-known statistical evaluation tools, for example, the determination of confidence intervals, the determination of p-values, Student's t-test, Mann-Whitney test, etc. Details can be found in Dowdy and Wearden, Statistics for Research, John Wiley and Sons, New York 1983. Preferred confidence intervals are at least 90%, at least 95%, at least 97%, at least 98%, or at least 99%. The p-values are preferably 0.1, 0.05, 0.01, 0.005, or 0.0001. More preferably, at least 60%, at least 70%, at least 80%, or at least 90% of the subjects of a population can be suitably identified by the method of the present invention.

As it is used herein, the term "cerebrovascular disease" refers to a clinical syndrome caused by the alteration of the blood supply to the brain, characterized by rapidly developing signs of an alteration of the overall cerebral function which last for more than 24 hours or lead to death. A cerebrovascular disease is usually known as apoplexy, given that it is the most common cerebrovascular disease. Cerebrovascular events according to the invention include transient ischemic attack (TIA), apoplexy, carotid artery disease, and amaurosis fugax. In a preferred embodiment, the cerebrovascular disease is apoplexy.

As it is used herein, the term "transient ischemic attack" or TIA refers to an episode of neurological dysfunction caused by ischemia (loss of blood flow either in the brain, the spinal cord, or the retina) without acute infarction (dead tissue). TIAs have the same underlying cause as apoplexies: a rupture of cerebral blood flow (CBF), and they are often referred to as mini-apoplexies or mega-apoplexies.

The term "apoplexy," also known as cerebrovascular accident (CVA), cerebrovascular incident (CVI), or stroke, is used herein to refer to the situation in which there is a lack of blood flow to the brain, giving rise to cell death. Ischemic apoplexy (also known as ischemic infarction) is the result of the lack of blood flow, as a consequence of an obstruction of a blood vessel that supplies blood flow to the brain. Hemorrhagic apoplexy (also known as intracerebral hemorrhage) is due to a hemorrhage. As a result of the apoplexy, the brain does not function properly. The signs and symptoms of apoplexy may include: inability to move or lack of feeling on one side of the body, comprehension or speaking issues, dizziness, loss of vision, among others. The signs and symptoms often appear immediately after the apoplexy has taken place. If the symptoms last for less than one or two hours, it is known as a transient ischemic attack (TIA).

The term "carotid artery disease," also known as "carotid artery stenosis" or "carotid stenosis," refers to a stricture or constriction of the inner surface (lumen) of the carotid artery, usually caused by atherosclerosis. Methods for determining carotid artery stenosis are known to one skilled in the art and include, without limitation, Doppler duplex ultrasound, arteriogram, computed axial tomography angiography (CTA), or magnetic resonance angiography (MRA).

As it is used herein, the term "amaurosis fugax" refers to a painless and transient monocular vision loss.

Step (i) of the prognostic method of the invention comprises determining the expression level of at least one gene selected from the group of genes shown in Table 1 in a biological sample from said subject or determining the expression level of at least one gene selected from the group of genes shown in Table 2 in a biological sample from said subject.

In a particular embodiment, the sample is a sample containing cells from the atherosclerotic plaque. In a more preferred embodiment, the sample is a lysate of entire plaques, and therefore contains material derived from all the cells forming part of the plaque. In a more preferred embodiment, the cells extracted from the atherosclerotic plaque are smooth muscle cells (SMCs). In an even more preferred embodiment, the smooth muscle cells are MYH11-positive and/or PECAM1-negative. The methods for obtaining and identifying the cells of interest have been described above and are likewise applicable to this method.

In a particular embodiment, the expression level of a gene is determined by means of RNA sequencing techniques.

In a particular embodiment, the prognostic method of the invention comprises determining the expression level of at least one gene from group 1, at least one gene from group 2, at least one gene from group 3, at least one gene from group 4, at least one gene from group 5, and/or at least one gene from group 6.

In another particular embodiment, the prognostic method of the invention comprises determining the expression level of all the genes from group 1.

In another particular embodiment, the prognostic method of the invention comprises determining the expression level of all the genes from group 2.

In another particular embodiment, the prognostic method of the invention comprises determining the expression level of all the genes from group 3.

In another particular embodiment, the prognostic method of the invention comprises determining the expression level of all the genes from group 4.

In another particular embodiment, the prognostic method of the invention comprises determining the expression level of all the genes from group 5.

In another particular embodiment, the prognostic method of the invention comprises determining the expression level of all the genes from group 6.

In another particular embodiment, the prognostic method of the invention comprises determining the expression level of all the genes from Table 2.

In another particular embodiment, the prognostic method of the invention comprises determining the expression level of all the genes from Table 3.

The methods for determining the expression levels of the gene of interest, particularly the levels of the genes included in Table 1 or in Table 2, have been described above in the context of the diagnostic method of the invention and are likewise applicable in the present method, including the limitations thereof.

In another particular embodiment, the prognostic method of the invention comprises determining the expression level of all the genes from group 1, all the genes from group 2, all the genes from group 3, all the genes from group 4, all the genes from group 5, and all the genes from group 6.

In another particular embodiment, the prognostic method of the invention comprises determining the expression level of all the genes from Table 1.

Step (ii) of the prognostic method of the invention comprises comparing the expression level obtained in (i) with a reference value.

In a particular embodiment, the reference value of a gene corresponds to the expression level of said gene in an atherosclerotic plaque cell sample from a subject who has stable atherosclerotic plaques.

In a particular embodiment, the reference value corresponds to the expression level of said gene in an atherosclerotic plaque cell sample from a subject who has not suffered from a cerebrovascular disease.

According to the prognostic method of the invention, if the expression level of the gene of interest 1-33 increases with respect to the reference value, and/or if the expression level of the gene of interest 34-93 decreases with respect to the reference value, then the subject shows a high probability of suffering from a cerebrovascular disease.

Alternatively, according to the prognostic method of the invention, wherein if the at least one gene determined in step (i) is one of genes 1-6 from Table 2 and its expression is increased with respect to the reference value, and/or if the at least one gene determined in step (i) is one of genes 7-11 from Table 2 and its expression is reduced with respect to the reference value, then the subject has a high probability of suffering from a cerebrovascular disease.

As it is used herein, "high probability" refers to the situation in which the subject shows at least 5%, at least 10%, at least 20%, at least 30%, at least 40%, at least 50%, at least 60%, at least 70%, at least 80%, at least 90%, at least 100% probabilities of having a cerebrovascular disease over time.

As it is used herein, "low probability" refers to the situation in which the subject shows at least 5%, at least 10%, at least 20%, at least 30%, at least 40%, at least 50%, at least 60%, at least 70%, at least 80%, at least 90%, at least 100% probabilities of not having a cerebrovascular disease over time.

The terms and limitations described above in relation to the diagnostic method of the invention are likewise applicable to this aspect.

### Method of treatment of a subject for removing or stabilizing the carotid atherosclerotic plaque

The presence of an unstable atherosclerotic plaque is associated with an increase in the risk of suffering from a cerebrovascular disease (Insull, W. Am. J. Med. 122 (1 suppl), S3-S14 (2009). Accordingly, changes in the expression levels of those genes that are markers of the presence of an unstable atherosclerotic plaque also allow selecting patients having unstable atherosclerotic plaque for a treatment to remove or stabilize said plaques.

Therefore, in a third aspect the invention relates to a method for removing or stabilizing the carotid atherosclerotic plaque in a subject which comprises
(i) determining the expression level of at least one gene selected from the group of genes shown in Table 1 or Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is treated for removing or stabilizing the carotid atherosclerotic plaque.

Alternatively, the invention relates to a compound suitable for removing or stabilizing the carotid atherosclerotic plaque in a subject for use in the treatment for carotid atherosclerotic plaque, which comprises
(i) determining the expression level of at least one gene selected from the group of genes shown in Table 1 or in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is treated for removing or stabilizing the carotid atherosclerotic plaque.

As it is used herein, "stabilizing the carotid atherosclerotic plaque" refers to reducing the possibility of the atherosclerotic plaque rupturing and/or becoming detached.

Step (i) of the method for removing the carotid atherosclerotic plaque comprises determining the expression level of at least one gene selected from the group of genes shown in Table 1 or Table 2 in a biological sample from said subject.

In a particular embodiment, the sample is a sample containing cells from the atherosclerotic plaque. In a more preferred embodiment, the sample is a lysate of entire plaques and therefore contains material derived from all the cells forming part of the plaque. In a more preferred embodiment, the cells extracted from the atherosclerotic plaque are smooth muscle cells (SMCs). In an even more preferred embodiment, the smooth muscle cells are MYH11-positive and PECAM1-negative. The methods for obtaining and identifying the cells of interest have been described above and are likewise applicable to this method.

In a particular embodiment, the expression level of a gene is determined by means of RNA sequencing techniques.

In a particular embodiment, the method for removing the carotid atherosclerotic plaque comprises determining the expression level of at least one gene from group 1, at least one gene from group 2, at least one gene from group 3, at least one gene from group 4, at least one gene from group 5, and/or at least one gene from group 6.

In another particular embodiment, the method for removing the carotid atherosclerotic plaque comprises determining the expression level of all the genes from group 1.

In another particular embodiment, the method for removing the carotid atherosclerotic plaque comprises determining the expression level of all the genes from group 2.

In another particular embodiment, the method for removing the carotid atherosclerotic plaque comprises determining the expression level of all the genes from group 3.

In another particular embodiment, the method for removing the carotid atherosclerotic plaque comprises determining the expression level of all the genes from group 4.

In another particular embodiment, the method for removing the carotid atherosclerotic plaque comprises determining the expression level of all the genes from group 5.

In another particular embodiment, the method for removing the carotid atherosclerotic plaque comprises determining the expression level of all the genes from group 6.

In another particular embodiment, the method for removing the carotid atherosclerotic plaque comprises determining the expression level of all the genes from Table 2.

In another particular embodiment, the method for removing the carotid atherosclerotic plaque comprises determining the expression level of all the genes from Table 3.

The methods for determining the expression levels of the gene of interest, particularly the levels of the genes included in Table 1 or Table 2, have been described above in the context of the diagnostic method of the invention and are likewise applicable in the present method, including the limitations thereof.

In another particular embodiment, the method for removing the carotid atherosclerotic plaque comprises determining the expression level of all the genes from group 1, all the genes from group 2, all the genes from group 3, all the genes from group 4, all the genes from group 5, and all the genes from group 6.

In another particular embodiment, the method for removing the carotid atherosclerotic plaque comprises determining the expression level of all the genes from Table 1 or of all the genes from Table 2.

In a particular embodiment, the reference value of a gene corresponds to the expression level of said gene in an atherosclerotic plaque cell sample from a subject who has stable atherosclerotic plaques.

In a particular embodiment, after applying treatment the response of the subject to therapeutic treatment is monitored through the detection of the identified biomarkers.

In a particular embodiment, the removal of the atherosclerotic plaque is performed by means of carotid endarterectomy.

As it is used herein, the term "carotid endarterectomy," or EAC, refers to a surgical procedure used to reduce the risk of apoplexy, to correct stenosis (stricture) in the common carotid artery or internal carotid artery. Endarterectomy involves removing material from inside an artery.

In a particular embodiment, statins are used as a treatment for stabilizing the carotid plaque.

As it is used herein, the term "statin" refers to a inhibitor of 3-hydroxy-3-methylglutaryl-coenzyme A (HMG-CoA) reductase, the enzyme that catalyzes the limiting step of cholesterol biosynthesis, and includes any known or newly synthesized or newly designed natural, synthetic, or semisynthetic statin or molecules related to statins; as it is used herein, the term "molecules related" to statins, refers to those molecules with lipid-lowering (cholesterol-lowering or triglyceride-lowering) capacity. Non-limiting illustrative examples of statins which can be used in the context of the present invention include atorvastatin, cerivastatin, fluvastatin, lovastatin, mevastatin, a monacolin (e.g., monacolin M, monacolin J, monacolin N, monacolin L, monacolin X, etc.), pitavastatin (also called itavastatin), pravastatin, rosuvastatin, simvastatin, etc., as well as combinations of any two or more statins, for example, monacolins, for example, monacolin M, monacolin J, monacolin N, monacolin L, monacolin X, etc., or any other combination of any two or more statins. Some statins can be in closed form (lactone) or in open form (hydroxy acid). Hydroxy acids (open form) can be prepared from the corresponding lactones by conventional hydrolysis, for example, with sodium hydroxide in methanol, sodium hydroxide in tetrahydrofuran-water, and the like.

In another particular embodiment, antiaggregants are used to stabilize the atherosclerotic plaque.

As it is used herein, "antiaggregant" or "antiplatelet drug" refers to a compound the main effect of which is to inhibit platelet aggregation and therefore the formation of thrombi or clots in arteries and veins. Non-limiting illustrative examples of antiaggregants are cyclooxygenase inhibitors, such as acetylsalicylic acid, sulfinpyrazone, triflusal, ditazol, indobufen; ADP receptor inhibitors such as ticlopidine, clopidogrel, prasugrel; glycoprotein IIa/IIIb or GPIIb-IIIa receptor antagonists, such as tigramin, eptifibatide, tirofiban, abciximab.

The terms and limitations described above in relation to the diagnostic method and the prognostic method of the invention are likewise applicable to this aspect.

### Method for selecting patients to be subjected to removal of the carotid atherosclerotic plaque

In another aspect, the invention relates to a method for selecting a patient susceptible to being treated with a therapy for removing or stabilizing the carotid atherosclerotic plaque, which comprises
(i) determining the expression level of at least one gene selected from the group of genes shown in Table 1 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is susceptible to being treated with a therapy for removing or stabilizing the carotid atherosclerotic plaque.

As it is used herein, the term "selecting" refers to the action of choosing a subject to subject him or her to a preventive or curative treatment for carotid atherosclerotic plaque, and therefore being able to prevent a cerebrovascular disease.

In a particular embodiment, the therapy for removing the carotid atherosclerotic plaque is carotid endarterectomy.

In a particular embodiment, the treatment for stabilizing the carotid atherosclerotic plaque is a treatment with statins or antiaggregants.

Step (i) of the method for selecting a patient comprises determining the expression level of at least one gene selected from the group of genes shown in Table 1 or from the group of genes shown in Table 2 in a biological sample from said subject.

In a particular embodiment, the sample is a sample containing cells from the atherosclerotic plaque. In a more preferred embodiment, the sample is a lysate of entire plaques and therefore contains material derived from all the cells forming part of the plaque. In a more preferred embodiment, the cells extracted from the atherosclerotic plaque are smooth muscle cells (SMCs). In an even more preferred embodiment, the smooth muscle cells are MYH11-positive and PECAM1-negative. The methods for obtaining and identifying the cells of interest have been described above and are likewise applicable to this method.

In a particular embodiment, the expression level of a gene is determined by means of RNA sequencing techniques.

In a particular embodiment, the method for selecting a patient comprises determining the expression level of at least one gene from group 1, at least one gene from group 2, at least one gene from group 3, at least one gene from group 4, at least one gene from group 5, and/or at least one gene from group 6.

In another particular embodiment, the method for selecting a patient comprises determining the expression level of all the genes from group 1.

In another particular embodiment, the method for selecting a patient comprises determining the expression level of all the genes from group 2.

In another particular embodiment, the method for selecting a patient comprises determining the expression level of all the genes from group 3.

In another particular embodiment, the method for selecting a patient comprises determining the expression level of all the genes from group 4.

In another particular embodiment, the method for selecting a patient comprises determining the expression level of all the genes from group 5.

In another particular embodiment, the method for selecting a patient comprises determining the expression level of all the genes from group 6.

In another particular embodiment, the method for selecting a patient comprises determining the expression level of all the genes from Table 2.

In another particular embodiment, the method for selecting a patient comprises determining the expression level of all the genes from Table 3.

The methods for determining the expression levels of the gene of interest, particularly the levels of the genes included in Table 1 or Table 2, have been described above in the context of the diagnostic method of the invention and are likewise applicable in the present method, including the limitations thereof.

In another particular embodiment, the method for selecting a patient comprises determining the expression level of all the genes from group 1, all the genes from group 2, all the genes from group 3, all the genes from group 4, all the genes from group 5, and all the genes from group 6.

In another particular embodiment, the method for selecting a patient comprises determining the expression level of all the genes from Table 1 or Table 2.

In a particular embodiment, the reference value of a gene corresponds to the expression level of said gene in an atherosclerotic plaque cell sample from a subject who has stable atherosclerotic plaques.

The terms and limitations described above in relation to the diagnostic method and the prognostic method of the invention are likewise applicable to this aspect.

### Kits of the invention

In another aspect, the invention relates to a kit comprising reagents suitable for determining the expression levels of at least one gene selected from the group of genes shown in Table 1 or of at least one gene selected from the group of genes shown in Table 2, and optionally reagents for determining the expression levels of one or more housekeeping genes.

In the context of the present invention, "kit" is understood as a product containing the different reagents required for carrying out the methods of the invention packaged such that it allows being transported and stored. The materials suitable for the packaging of the components of the kit include glass, plastic (polyethylene, polypropylene, polycarbonate, and the like), bottles, vials, paper, sachets, and the like. Furthermore, the kits of the invention can contain instructions for the simultaneous, sequential, or separate use of the different components that are in the kit. Said instructions can be in the form of printed material or in the form of an electronic medium capable of storing instructions such that they can be read by a subject, such as electronic storage media (magnetic disks, tapes, and the like), optical media (CD-ROM, DVD), and the like. The media may additionally or alternatively contain Internet addresses providing said instructions.

In a particular embodiment, the kit comprises reagents suitable for determining the expression level of at least 1 gene, at least 2 genes, at least 3 genes, at least 4 genes, at least 5 genes, at least 6 genes, at least 7 genes, at least 8 genes, at least 9 genes, at least 10 genes, at least 11 genes, at least 12 genes, at least 13 genes, at least 14 genes, at least 15 genes, at least 16 genes, at least 17 genes, at least 18 genes, at least 19 genes, at least 20 genes, at least 21 genes, at least 22 genes, at least 23 genes, at least 24 genes, at least 25 genes, at least 26 genes, at least 27 genes, at least 28 genes, at least 29 genes, at least 30 genes, at least 31 genes, at least 32 genes, at least 33 genes, at least 34 genes, at least 35 genes, at least 36 genes, at least 37 genes, at least 38 genes, at least 39 genes, at least 40 genes, at least 41 genes, at least 42 genes, at least 43 genes, at least 44 genes, at least 45 genes, at least 46 genes, at least 47 genes, at least 48 genes, at least 49 genes, at least 50 genes, at least 51 genes, at least 52 genes, at least 53 genes, at least 54 genes, at least 55 genes, at least 56 genes, at least 57 genes, at least 58 genes, at least 59 genes, at least 60 genes, at least 61 genes, at least 62 genes, at least 63 genes, at least 64 genes, at least 65 genes, at least 66 genes, at least 67 genes, at least 68 genes, at least 69 genes, at least 70 genes, at least 71 genes, at least 72 genes, at least 73 genes, at least 74 genes, at least 75 genes, at least 76 genes, at least 77 genes, at least 78 genes, at least 79 genes, at least 80 genes, at least 81 genes, at least 82 genes, at least 83 genes, at least 84 genes, at least 85 genes, at least 86 genes, at least 87 genes, at least 88 genes, at least 89 genes, at least 90 genes, at least 91 genes, at least 92 genes, or of the 93 genes included in Table 1.

In a particular embodiment, the kit comprises reagents suitable for determining the expression level of at least 1 gene, at least 2 genes, at least 3 genes, at least 4 genes, at least 5 genes, at least 6 genes, at least 7 genes, at least 8 genes, at least 9 genes, at least 10 genes, or at least 11 genes included in Table 2.

According to the present invention, the kit comprises reagents specific for determining any combination of genes 1-93 from Table 1.

According to the present invention, the kit comprises reagents specific for determining any combination of genes 1-11 from Table 2.

In a particular embodiment, the kit of the invention comprises reagents suitable for determining genes 1-93 from Table 1.

According to the present invention, the kit comprises reagents specific for determining all genes 1-11 from Table 2.

In another particular embodiment, the kit of the invention comprises determining the expression level of at least one gene from group 1, at least one gene from group 2, at least one gene from group 3, at least one gene from group 4, at least one gene from group 5, and/or at least one gene from group 6.

In a particular embodiment, the kit comprises reagents suitable for determining the expression level of at least one gene selected from group 1 consisting of ADAMTS7, ANXA3, BMP2, CLEC3B, CRHR1, DDR2, DHRS3, EPHA4, EPHB2, ESM1, FLRT3, FRAS1 GATA2, ID1, ID4, ITGA7, KCNJ8, KLF5, MYO18B, NPPC, NRCAM, NRK, PI16, PRICKLE1, PTHLH, PTPRB, RDH10, SEMA3G, SGCD, SMAD9, SOCS3, SOX17, TBX18, XDH, GJA5, TXNIP, SYNE3, KCNE4, and SDPR.

In another particular embodiment, the kit comprises reagents suitable for determining the expression level of at least one gene selected from group 2 consisting of ADAMTS7, BAIAP2, BMP2, CA12, CLEC3B, CHODL, DDR2, GATA2, ID1, ID4, IL11, ITGA7, MGST1, NPPC, PPP1R1C, PTHLH, RNF43, SOCS3, TMTC2, and TNFAIP8L3.

In another particular embodiment, the kit comprises reagents suitable for determining the expression level of at least one gene selected from group 3 consisting of ABCG2, ANXA3, APOA1, BMP2, C10orf10, CHRNA5, CRHR1, G0S2, HAPLN1, HLA-B, ID1, ID4, KCNJ8, NRCAM, PTHLH, RGS2, RNF43, SOX17, ST6GALNAC5, XDH, and FRMD3.

In another particular embodiment, the kit comprises reagents suitable for determining the expression level of at least one gene selected from group 4 consisting of ADAMTS7, APOA1, BAIAP2, BMP2, CHODL, DDR2, DHRS3, DKK2, EPHA4, EPHB2, FLRT3, GATA2, HLA-B, ID1, ID4, IL17RD, KLF5, LRRC4C, NPPC, NRCAM, PI16, PRICKLE1, PTHLH, RGS2, RND1, RNF43, SEMA3G, SLITRK4, SMAD9, SOCS3, SOX17, TBX18, XDH, FAM27A, RP11-351M8.1, ZSCAN31, ANXA10, C19orf33, CCL5, DIRAS3, GULP1, IL20RB, MYCT1, and PFKFB4.

In another particular embodiment, the kit comprises reagents suitable for determining the expression level of at least one gene selected from group 5 consisting of BMP2, NRCAM, CD248, ANXA3, DAPK2, DDR2, GATA2, SOX17, ID1, APOA1, FLRT3, SEMA3G, ITGA7, and EPHA4.

In another particular embodiment, the kit comprises reagents suitable for determining the expression level of at least one gene selected from group 6 consisting of ABCG2, APOA1, BAIAP2, BMP2, CADM3, DAPK2, DMRTA1, EPHA4, EPHB2, ESM1, FGF11, FLRT3, GLDC, HLA-B, ID1, IL11, ITGA7, KCNJ8, MCHR1, MGST1, NPPC, NRXN2, PDK1, PTHLH, RND1, RNF43, SEMA3G, TBX18, XDH, PPP1R3C, and CHRNE.

In another particular embodiment, the kit comprises reagents for determining the expression level of all the genes from group 1, specifically of ADAMTS7, ANXA3, BMP2, CLEC3B, CRHR1, DDR2, DHRS3, EPHA4, EPHB2, ESM1, FLRT3, FRAS1 GATA2, ID1, ID4, ITGA7, KCNJ8, KLF5, MYO18B, NPPC, NRCAM, NRK, PI16, PRICKLE1, PTHLH, PTPRB, RDH10, SEMA3G, SGCD, SMAD9, SOCS3, SOX17, TBX18, XDH, GJA5, TXNIP, SYNE3, KCNE4, and SDPR.

In another particular embodiment, the kit comprises reagents for determining the expression level of all the genes from group 2, specifically of ADAMTS7, BAIAP2, BMP2, CA12, CLEC3B, CHODL, DDR2, GATA2, ID1, ID4, IL11, ITGA7, MGST1, NPPC, PPP1R1C, PTHLH, RNF43, SOCS3, TMTC2, and TNFAIP8L3.

In another particular embodiment, the kit comprises reagents for determining the expression level of all the genes from group 3, specifically of ABCG2, ANXA3, APOA1, BMP2, C10orf10, CHRNA5, CRHR1, G0S2, HAPLN1, HLA-B, ID1, ID4, KCNJ8, NRCAM, PTHLH, RGS2, RNF43, SOX17, ST6GALNAC5, XDH, and FRMD3.

In another particular embodiment, the kit comprises reagents for determining the expression level of all the genes from group 4, specifically of ADAMTS7, APOA1, BAIAP2, BMP2, CHODL, DDR2, DHRS3, DKK2, EPHA4, EPHB2, FLRT3, GATA2, HLA-B, ID1, ID4, IL17RD, KLF5, LRRC4C, NPPC, NRCAM, PI16, PRICKLE1, PTHLH, RGS2, RND1, RNF43, SEMA3G, SLITRK4, SMAD9, SOCS3, SOX17, TBX18, XDH, FAM27A, RP11-351M8.1, ZSCAN31, ANXA10, C19orf33, CCL5, DIRAS3, GULP1, IL20RB, MYCT1, and PFKFB4.

In another particular embodiment, the kit comprises reagents for determining the expression level of all the genes from group 5, specifically of BMP2, NRCAM, CD248, ANXA3, DAPK2, DDR2, GATA2, SOX17, ID1, APOA1, FLRT3, SEMA3G, ITGA7, and EPHA4.

In another particular embodiment, the kit comprises reagents suitable for determining the expression level of all the genes from group 6, specifically of ABCG2, APOA1, BAIAP2, BMP2, CADM3, DAPK2, DMRTA1, EPHA4, EPHB2, ESM1, FGF11, FLRT3, GLDC, HLA-B, ID1, IL11, ITGA7, KCNJ8, MCHR1, MGST1, NPPC, NRXN2, PDK1, PTHLH, RND1, RNF43, SEMA3G, TBX18, XDH, PPP1R3C, and CHRNE.

In another particular embodiment, the kit comprises reagents suitable for determining the expression level of all the genes from Table 2 consisting of genes DENND5A, LPIN2, MARCH8, PLAU, SCARB1, SLC2A3, REEP2, IFI27L1, MMP16, PLA2G4A, and SLC26A2

In another particular embodiment, the kit comprises reagents suitable for determining the expression level of all the genes from Table 3 consisting of genes ST6GALNAC5, ITGA7, MOCOS, CA12, SYNE3, PFKFB4, ADAMTS7, PDK1, TBX18, DDR2, SOX17, IL17RD, APOA1, ID1, NRK, PTPRB, DHRS3, KLF5, FRMD3, PI16, ABCG2, and FRAS1.

In another particular embodiment, the kit comprises reagents suitable for determining the expression level of all the genes from group 1, all the genes from group 2, all the genes from group 3, all the genes from group 4, all the genes from group 5, and all the genes from group 6.

In a particular embodiment of the kits of the invention, said kits optionally comprise reagents for determining the expression levels of one or more housekeeping genes.

In another embodiment, the kit according to the present invention further comprises reagents suitable for determining the expression levels of genes that are expressed specifically or are not expressed in smooth muscle cell populations, specifically MYH11 and PECAM1.

In a preferred embodiment, the reagents suitable for determining the expression levels of one or more genes comprise at least 10%, at least 20%, at least 30%, at least 40%, at least 50%, at least 60%, at least 70%, at least 80%, at least 90%, or at least 100% of the total amount of the reagents suitable for determining the expression levels of genes making up the kit.

In additional embodiments, the reagents suitable for determining the expression levels of one or more genes comprise at least 55%, at least 60%, at least 65%, at least 70%, at least 75%, at least 80%, at least 90%, at least 95%, at least 96%, at least 97%, at least 98%, or at least 99% of the total amount of reagents making up the kit.

In that sense, in the particular case of kits comprising the reagents for determining the expression levels of the genes from group 1 ADAMTS7, ANXA3, BMP2, CLEC3B, CRHR1, DDR2, DHRS3, EPHA4, EPHB2, ESM1, FLRT3, FRAS1 GATA2, ID1, ID4, ITGA7, KCNJ8, KLF5, MYO18B, NPPC, NRCAM, NRK, PI16, PRICKLE1, PTHLH, PTPRB, RDH10, SEMA3G, SGCD, SMAD9, SOCS3, SOX17, TBX18, XDH, GJA5, TXNIP, SYNE3, KCNE4, and SDPR, the reagents specific for said genes (for example, probes that are capable of hybridizing under stringent conditions with genes ADAMTS7, ANXA3, BMP2, CLEC3B, CRHR1, DDR2, DHRS3, EPHA4, EPHB2, ESM1, FLRT3, FRAS1 GATA2, ID1, ID4, ITGA7, KCNJ8, KLF5, MYO18B, NPPC, NRCAM, NRK, PI16, PRICKLE1, PTHLH, PTPRB, RDH10, SEMA3G, SGCD, SMAD9, SOCS3, SOX17, TBX18, XDH, GJA5, TXNIP, SYNE3, KCNE4, and SDPR) comprise at least 10%, at least 20%, at least 30%, at least 40%, at least 50%, at least 60%, at least 70%, at least 80%, at least 90%, or at least 100% of the probes present in the kit.

The expression "reagent that allows determining the expression level of a gene" means a compound or set of compounds that allows determining the expression level of a gene, both by means of determining the level of mRNA and by means of determining the level of protein. Therefore, the reagents of the first type include probes capable of hybridizing specifically with the mRNA encoded by said genes. The reagents of the second type include compounds that bind specifically to the proteins encoded by the marker genes, and antibodies are preferably included, although they can be specific aptamers.

In a particular embodiment of the kit of the invention, the reagents of the kit are nucleic acids that are capable of detecting specifically the level of mRNA of the aforementioned genes and/or the level of proteins encoded by one or more of the aforementioned genes. The nucleic acids capable of hybridizing specifically with the aforementioned genes can be one or more pairs of oligonucleotide primers for the specific amplification of fragments of the mRNAs (or the corresponding cDNAs) of said genes.

In a preferred embodiment, the first component of the kit of the invention comprises a probe which can hybridize specifically with the aforementioned genes.

As it is used herein, the term "hybridizing specifically" refers to the conditions that allow the hybridization of two polynucleotides under highly stringent conditions or moderately stringent conditions.

The "stringency" of the hybridization reactions can be readily determined by one skilled in the art, and it is generally an empirical calculation dependent on the length of the probe, the wash temperature, and the salt concentration. Generally, longer probes require higher temperatures for suitable hybridization, whereas shorter probes require lower temperatures. Hybridization generally depends on the capacity of denatured DNA to rehybridize when complementary strands are present in an environment that is below the melting temperature thereof. The higher the degree of desired homology between the probe and the hybridizable sequence, the higher the relative temperature that can be used is. As a result, it is deduced that higher relative temperatures would tend to make reaction conditions more stringent, whereas lower temperatures would make them less stringent. For additional explanations and details about hybridization reaction stringency, see Ausubel et al., Current Protocols in Molecular Biology, Wiley Interscience Publishers, (1995).

As defined herein, "stringent conditions" or "highly stringent conditions" generally: (1) use a low ionic strength and high temperature for the wash, for example, 0.015 M sodium chloride/0.0015 M sodium citrate/0.1% sodium dodecyl sulfate at 50°C; (2) use a denaturing agent during hybridization, such as formamide, for example, 50% formamide (v/v) with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C; or (3) use 50% formamide, 5xSSC (0.75 M NaCl, 0.075 M sodium citrate), 50 mM sodium phosphate (pH 6.8), 0.1% sodium pyrophosphate, 5x Denhardt's solution, sonicated salmon sperm DNA (50 mg/ml), 0.1% SDS, and 10% dextran sulfate at 42°C, with washes at 42°C in 0.2xSSC (sodium chloride/sodium citrate) and 50% formamide, followed by a high stringency wash consisting of 0.1xSSC containing EDTA at 55°C.

"Moderately stringent conditions" can be identified as described by Sambrook et al., Molecular Cloning: A Laboratory Manual, New York: Cold Spring Harbor Press, 1989, and include the use of a washing solution and hybridization conditions (for example, temperature, ionic strength, and % of SDS) that are less stringent that those described above. An example of moderately stringent conditions is incubation overnight at 37°C in a solution comprising: 20% formamide, 5xSSC (150 mM NaCl, 15 mM sodium citrate), 50 mM sodium phosphate (pH 7.6), 5x Denhardt's solution, 10% dextran sulfate, and 20 mg/ml of denatured sheared salmon sperm DNA, followed by washing the filters in 1xSSC at about 37-50°C. One skilled in the art will recognize how to adjust the temperature, ionic strength, etc., as required in order to allow for factors such as length of the probe and the like.

In the event that the expression levels of several of the genes identified in the present invention are determined at the same time, it is useful to include the probes for all the genes the expression of which is to be determined in microarray hybridization.

The microarrays comprise a plurality of nucleic acids which are spatially distributed and stably associated with a support (for example, a biochip). The nucleic acids have a sequence that is complementary to particular subsequences of genes the expression of which is to be detected; they are therefore capable of hybridizing with said nucleic acids. In the methods of the invention, a microarray comprising a set of nucleic acids is placed in contact with a preparation of nucleic acids isolated from the patient under study. The microarray with the preparation of nucleic acids is incubated under conditions suitable for hybridization. Then, after removing the nucleic acids not retained by the support, the hybridization pattern is detected, which provides information about the genetic profile of the analyzed sample. Although the microarrays are capable of providing both qualitative and quantitative information of the nucleic acids present in a sample, the invention requires using arrays and methodologies capable of providing quantitative information.

The invention provides for a range of arrays in relation to the type of probes and in relation to the type of support used. The probes included in the arrays that are capable of hybridizing with the nucleic acids can be nucleic acids or analogues thereof which maintain the hybridization capacity, such as, for example, nucleic acids in which the phosphodiester bond has been replaced with a methylimine, methylphosphonate, phosphoramidate, guanidine, phosphorothioate bond, and the like, nucleic acids in which the ribose of the nucleotides is replaced with another hexose, peptide nucleic acids (PNAs). The length of the probes can be from 5 to 50 nucleotides, and preferably 7, 10, 15, 20, 25, 30, 35, 40, 45, 50, 55, 60, 65, 70, 75, 100 nucleotides, and they vary in the range of 10 to 1000 nucleotides, preferably in the range of 15 to 150 nucleotides, more preferably in the range of 15 to 100 nucleotides, and they can be single-stranded or double-stranded nucleic acids. The array may contain all the specific probes of a given mRNA of a certain length or it may contain probes selected from different regions of an mRNA. Each probe is assayed in parallel with a probe with a changed base, preferably in a central region of the probe. The array is placed in contact with a sample containing the nucleic acids with sequences complementary to the probes of the array, and the hybridization signal resulting from hybridization with each of the probes and the corresponding hybridization controls is determined. Those probes in which a greater difference is observed between the hybridization signal resulting from hybridization with the probe and its hybridization control are selected. The optimization process may include a second optimization round in which the hybridization array is hybridized with a sample that does not contain sequences that are complementary to the probes of the array. After the second selection round, the probes with hybridization signals that are lower than the threshold will be selected. Therefore, the probes which pass both controls, i.e., those that show a minimum level of non-specific hybridization and a maximum level of specific hybridization with the target nucleic acid, are selected.

The selection of the probes specific for the different target genes is carried out such that they bind specifically to the target nucleic acid with minimal hybridization with unrelated genes. However, there are probes that are 20 nucleotides long that are not unique for a given mRNA. Therefore, the probes targeting said sequences will show cross-hybridization with identical sequences that appear in the mRNA of unrelated genes. Furthermore, there are probes that do not hybridize specifically with the target genes under the conditions used (due to secondary structures or interaction with the array substrate). This type of probe cannot be included in the array. Therefore, one skilled in the art will observe that the probes to be incorporated in a given array must be optimized before being incorporated in the array. Probe optimization is generally performed by means of generating an array containing a plurality of probes targeting the different regions of a certain target polynucleotide. This array is placed in contact first with a sample containing the target nucleic acid in an isolated form and then with a complex mixture of nucleic acids. The hybridization probes showing a very specific hybridization with the target nucleic acid but little or no hybridization with the complex sample are thereby selected to be incorporated in the arrays of the invention. Furthermore, it is possible to include in the array hybridization controls for each of the probes to be studied. In a preferred embodiment, the hybridization controls contain an altered position in the central region of the probe. In the event that high levels of hybridization between the probe under study and its hybridization control are observed, the probe is not included in the array.

The microarrays of the invention contain not only probes specific for the polynucleotides which indicates a given physiopathological situation, but which also contain a series of control probes, which can be of three types: normalization controls, expression level controls, and hybridization controls.

Normalization controls are oligonucleotides which are perfectly complementary to the labeled reference sequences which are added to the preparation of nucleic acids to be analyzed. The signals derived from the normalization controls after hybridization provide an indication of the variations in hybridization conditions, the intensity of the marker, detection efficiency, and another series of factors which can give rise to a variation in the hybridization signal between different microarrays. The signals detected from the remaining probes of the array are preferably divided by the signal emitted by the control probes, thereby normalizing the measurements. Virtually any probe can be used as a normalization control. However, hybridization efficiency is known to vary depending on nucleotide composition and probe length. Therefore, the preferred normalization probes are those which represent the mean length of the probes present in the array, although they may be selected such that they include a range of lengths which reflect the remaining probes present in the array. Normalization probes can be designed such that they reflect the mean composition of the nucleotides of the remaining probes present in the array. A limited number of normalization probes is preferably selected such that they suitably hybridize, i.e., they do not have a secondary structure and they have no sequence similarity with any of the probes of the array that is used. The normalization probes can be located in any position of the array or in several positions of the array for effective control of variations in hybridization efficiency in relation to the structure of the array. The normalization controls are preferably located in the corners of the array and/or in the center thereof.

Expression level controls are probes which hybridize specifically with genes that are constitutively expressed in the sample being analyzed. Expression level controls are designed to control the physiological state and metabolic activity of the cell. The study of the covariance of the expression level control of the target nucleic acid indicates if the variations in the expression levels are due to changes in expression levels or due to changes in the overall transcription rate in the cell or in its general metabolic activity. In that sense, in the case of cells having deficiencies in a given metabolite essential for cell viability, a decrease both in the expression levels of the target gene and in the expression levels of the control is expected to be observed. On the other hand, if an increase in the expression of the target gene and of the control genes is observed, it is probably due to an increase in the metabolic activity of the cell and not due to a differential increase in the expression of the target gene. Probes suitable for use as an expression control correspond to constitutively expressed genes, such as the genes encoding proteins which perform essential cell functions, such as β-2-microglobulin, ubiquitin, 18S ribosomal protein, cyclophilin A, transferrin receptor, actin, GAPDH, tyrosine 3-monooxygenase/tryptophan 5-monooxygenase activation protein (YWHAZ), and beta-actin.

Hybridization controls can be included both for the probes targeting the target genes and for the probes targeting the expression level or normalization controls. Error controls are oligonucleotide probes identical to the probes targeting the target genes, but which contain mutations in one or more nucleotides, i.e., they contain nucleotides in certain positions that do not hybridize with the corresponding nucleotide in the target gene. Hybridization controls are selected such that, by applying suitable hybridization conditions, the target gene must hybridize with the specific probe, but not with the hybridization control or doing so with a reduced efficiency. The hybridization controls preferably contain one or more modified positions in the center of the probe. The hybridization controls therefore provide an indication of the degree of non-specific hybridization or of cross-hybridization with a nucleic acid in the sample to a probe other than the one containing the exactly complementary sequence.

The arrays of the invention may also contain amplification controls and sample preparation controls which are probes complementary to subsequences of selected control genes because they normally do not appear in the biological sample under study, such as probes for bacterial genes. The RNA sample is supplemented with a known amount of a nucleic acid hybridizing with the selected control probe. The determination of the hybridization of said probe indicates the degree of recovery of the nucleic acids during preparation, as well as an estimate of the alteration caused in the nucleic acids during sample processing.

Once a set of probes showing suitable specificity and a set of control probes are provided, the latter are arranged in the array in a known position such that after the hybridization and detection steps, it is possible to establish a correlation between a positive hybridization signal and the particular gene from the coordinates of the array in which the positive hybridization signal is detected.

The microarrays can be high-density arrays, with thousands of oligonucleotides by means of photolithographic *in situ* synthesis methods (Fodor et al., 1991, Science, 767-773). This type of probe is usually redundant, i.e., it includes several probes for each mRNA to be detected. In a preferred embodiment, the arrays are low-density arrays, or LDAs, containing fewer than 10,000 probes per square centimeter. In said low-density arrays, the different probes are applied by hand with the aid of a pipette in different locations on a solid support (for example, a glass surface, a membrane). The supports used to fix the probes can be obtained from a wide range of materials, such as plastic, ceramic, metals, gels, membranes, glass, and the like. The microarrays can be obtained using any methodology known to one skilled in the art.

After hybridization, where the unhybridized nucleic acid is capable of emitting a signal in the detection step, a washing step is required to remove said unhybridized nucleic acid. The washing step is carried out using methods and solutions known to one skilled in the art.

In the event that the labeling in the nucleic acid is not directly detectable, it is possible to connect the microarray comprising the target nucleic acids bound to the array with the other components of the system required for causing the reaction giving rise to a detectable signal. For example, if the target nucleic acids are labeled with biotin, the array is placed in contact with streptavidin conjugated with a fluorescent reagent under conditions suitable for the binding between biotin and streptavidin to take place. After incubating the microarray with the system for generating the detectable signal, a washing step must be carried out to remove all the molecules that have bound in a non-specific manner to the array. The washing conditions will be determined by one skilled in the art using suitable conditions according to the system for generating the detectable signal that are well known to one skilled in the art.

The resulting hybridization pattern can be seen or detected in different ways; said detection is determined by the type of system used in the microarray. Therefore, hybridization pattern detection can be carried out by means of scintillation count, autoradiograph, determination of a fluorescent signal, calorimetric determinations, detection of a light signal, etc.

After hybridization and possible subsequent washing and treatment processes, the hybridization pattern is detected and quantified, whereby the signal corresponding to each point of hybridization in the array is compared with a reference value corresponding to the signal emitted by a known number of terminally labeled nucleic acids for the purpose of thereby obtaining an absolute value of the number of copies of each nucleic acid hybridized at a certain point of the microarray.

In the event that the expression levels of the genes according to the present invention are determined by means of measuring the levels of the polypeptide or polypeptides encoded by said gene or genes, the kits according to the present invention comprise reagents that are capable of binding specifically to said polypeptide or polypeptides.

To that end, the antibody arrays such as those described by De Wildt et al. (2000) Nat. Biotechnol. 18:989-994; Lueking et al. (1999) Anal. Biochem. 270:103-111; Ge et al. (2000) Nucleic Acids Res. 28, e3, I-VII; MacBeath and Schreiber (2000) Science 289:1760-1763, in patent documents WO 01/40803 and WO 99/51773A1, are useful. The antibodies of the array include any immunological agent capable of high affinity ligand binding, including IgG, IgM, IgA, IgD, and IgE, as well as antibody-like molecules having an antigen binding site, such as Fab', Fab, F(ab')2, single-domain antibodies or sdABs, Fv, scFv, and the like. The techniques for preparing said antibodies are well known to one skilled in the art and include the methods described by Ausubel et al. (Current Protocols in Molecular Biology, eds. Ausubel et al., John Wiley & Sons (1992)).

The antibodies of the array can be applied at high speed, for example, by means of commercially available robotic systems (for example, those produced by Genetics Microsystems or Biorobotics). The array substrate can be nitrocellulose, plastic, glass, or it can be made of a porous material, such as acrylamide, agarose, or another polymer, for example.

In another embodiment, it is possible to use cells that produce the antibodies specific for detecting the proteins of the invention by means of the culture thereof in array filters. After inducing expression of the antibodies, the latter are immobilized in the filter in the position of the array where the producing cell was located. An antibody array can be placed in contact with a labeled target, and the level of target-to-immobilized antibody binding can be determined. If the target is not labeled, a sandwich-type assay can be used, in which a second labeled antibody specific for the polypeptide binding to the polypeptide immobilized in the support is used. The quantification of the amount of polypeptide present in the sample at each point of the array can be stored in a database as an expression profile. The antibody array can be produced in duplicate and used to compare the binding profiles of two different samples.

In another aspect, the invention relates to the use of a kit of the invention for diagnosing unstable atherosclerotic plaques in a subject, for determining the probability of a subject suffering from a cerebrovascular disease, for removing the carotid atherosclerotic plaque, or for selecting a patient susceptible to being treated with a therapy for removing the carotid atherosclerotic plaque.

The terms and limitations described above in relation to the diagnostic method and the prognostic method of the invention are likewise applicable to this aspect.

The invention is described below by means of the following examples, which must be considered merely illustrative and by no means limiting of the scope of the present invention.

### EXAMPLES

### Materials and methods

### Selection of patients

Patients who had been subjected to carotid endarterectomy in Hospital Universitario of Basurto were selected to be included in the current study based on clinical parameters (age, symptoms, having no other clinical manifestations). All the patients were evaluated before and after the operation by a neuropsychology specialist. Radiology tests were completed to evaluate the degree of stenosis using Doppler ultrasound equipment. Only those patients complying with all the required parameters were finally included in the study (7A compared to 7S). A carotid tissue sample was taken after surgery and immediately shipped to the laboratory for cell isolation.

### Experimental strategy to differentiate the SMCs isolated from carotid plaques of symptomatic and asymptomatic patients by means of the RNAseq technology.

The study was designed such that the maximum power for detecting differentially expressed genes (DEGs) and differentially expressed isoforms (DEIs) was obtained, and such that complementary functional enrichment analysis and network analysis were performed. The two main factors to be taken into account in transcriptomic studies with RNAseq are the number of replicates and sequencing depth. Therefore, sequencing was performed with a depth to obtain about 60 million reads per sample, which allowed reaching a reliable differential expression detection power. Sequencing with this depth would allow identifying differential expression with a maximum power in 5 replicates, even in those genes with low expression levels. Furthermore, the variability in RNAseq analyses is reduced since the study is performed in a cell type (i.e., SMCs) compared with analysis in the entire plaque, which is heterogeneous and made up of different cell types, such as SMCs, endothelial cells, and macrophages. Accordingly, 14 (7 symptomatic and 7 asymptomatic) patients selected for an endarterectomy by the committee of medical experts of Hospital Universitario of Basurto, who evaluated the clinical data of the patients (Table 3), were selected to study the role of SMCs in the development of unstable carotid plaque. MRI and cervical duplex tests were performed in all the patients by the radiology department in the hospital. Patients with stenosis >70% and diagnosed as symptomatic (S) and patients with stenosis >80% c and without any symptom (A) were included in the study. Additional parameters required in this study were defined as: (1) age (70 years±12); (2) absence of other medical conditions; (3) absence of contralateral plaque; and (4) absence of other causes of CVA, such as a cardioembolic origin. During the recruitment period, several patients were excluded from the study for having at least one of the following problems: (1) patients who showed signs of dementia according to the mini-mental state examination (MMSE) test; (2) patients with psychological impairments due to drugs or brain surgery; (3) patients who presented medical or surgical complications that may have altered their intellectual capacity and/or the neuroimaging test; and (4) patients who were unable to perform the neuropsychological test due to a communication difficulty (i.e., vision problem).

### Smooth muscle cell culture and RNA purification

Fresh carotid artery tissue samples from symptomatic and asymptomatic patients were processed, being cut into 2-3 mm sized fragments which were digested with collagenase type I (Life Technologies, Carlsbad, CA, USA) for isolating and additionally culturing SMCs. First a 3-hour digestion at a concentration of 300 U/ml of collagenase type I was performed. Then, the digested tissue was subjected to digestion overnight with 220 U/ml of collagenase type I. The digested tissue was filtered by means of a 100 µm nylon Falcon™ Cell Strainer filter to remove the undigested material, and the cells were seeded in plates in medium specific for the growth of human SMCs referred to as M231 (Life Technologies, Carlsbad, CA, USA) complemented with SMC growth factors, such as 20 ng/ml of IGF-1, 2 ng/ml of EGFB, 0.5 ng/ml of EGF, 5 ng/ml of heparin, 0.2 µg/ml of BSA, and 5% fetal bovine serum (Sigma, St Louis, MO, USA). First-passage cells were selected for RNA extraction. Quality control of the pure SMC culture was performed by means of identifying the presence of specific SMC markers (MYH11) and the absence of an endothelial marker (PECAM1). SMC RNA extraction and purification was performed with the reagent TRIzol™ according to the manufacturer's instructions. Then the RNA was purified with the total RNA isolation kit RNeasy (QIAGEN, Valencia, CA, USA) following the instructions recommended by the manufacturer. RNA integrity (RIN>9) was analyzed using RNA 6000 Nano Chips in the Agilent 2100 bioanalyzer (Agilent Technologies, Palo Alto, CA, USA) .

### Western blot

Cell lysis was performed with RIPA lysis buffer (150 mM Tris HCl, 150 mM NaCl, 0.5% deoxycholate, 0.1% SDS, 1% NP-40) for 30 minutes at 4°C followed by centrifugation at 20,000*xg* for 10 minutes. The cell lysates were subjected to 6% or 12% SDS-PAGE gels. The proteins were transferred to the PVDF membrane. Immunodetections were performed with the following primary antibodies: anti-smooth muscle myosin heavy chain 11 antibody (ab82541, Abcam, Cambridge, RU) and smooth muscle actin polyclonal antibody (23081-1-AP, ProteinTech), anti-CD31 antibody (PECAM1-EPR3094 ProteinTech, Chicago, USA). The proteins were detected with HRP substrate detection reagent by means of Immobilon™ Western blot chemiluminescence (Millipore, Billerica, MA, USA), and they were viewed with the ChemiDoc™XRS imaging system (Bio-Rad, Richmond, CA, USA). The anti-glyceraldehyde-3-phosphate dehydrogenase antibody (MAB374, Millipore, Billerica, MA, USA) was used as an internal control for normalization. Data analysis was performed using Lab™ software (Bio-Rad, Richmond, CA, USA).

### Microscopy

The cells were grown on round glass slides (Thermo Scientific) followed by fixing in ice-cooled methanol. The samples were then blocked in 3% PBS/BSA w/v for 30 minutes and stained with anti-smooth muscle myosin heavy chain 11 antibody for 1 hour at room temperature. The cells were washed with PBS and incubated with Alexa Fluor®488 rabbit anti-goat IgG (H+L) secondary antibody for 45 minutes at room temperature in the dark, and the DNA was counterstained with 4',6-diamino-2-phenylindole (DAPI). After three washes, the slides were mounted in Fluoromount™ aqueous mounting medium (Sigma-Aldrich, St Louis, MO, USA). Image acquisition was performed using a Super-resolution Leica TCS STED CW SP8 microscope with a 40x lens and recording optical sections that were 0.3 µm each. Image analysis was performed using the ImageJ software (National Health Institute, USA).

### RNA library

Ribosomal RNA was removed with the Ribo-Zero kit for removing rRNA. Libraries were generated with the TruSeq single-stranded total RNA library preparation kit following the manufacturer's recommendations. It started from 2 µg of total RNA libraries (RIN>9), which were sequenced using a HiSeq2500 instrument (Illumina Inc, San Diego, CA, USA). The paired-end sequencing reads were taken with a read length of about 100 bp performed in 14 samples. The estimated coverage was about 60 million reads per sample (3 lanes). Library generation and RNA sequencing were performed at Sistemas Genómicos S.L. (Valencia, Spain) following the manufacturer's instructions.

### RNA transcriptomic analysis

The quality control of unprocessed data was performed using the FastQC tool. The unprocessed paired-end reads were mapped against the human genome provided by the Ensembl database (version GRchr37/hg19) using the tophat2 algorithm (Kim, D., et al. Genome Biol 14, 2013). The reads having insufficient quality (phred score <10) were eliminated using Picard Tools software (version 1.129). GC distribution (i.e., the guanine and cytosine bp ratio throughout the reads), which must have a desired distribution of between 40-60%, was evaluated in this step. Then, the distribution of duplicates (a sequencing quality indicator) was evaluated to confirm that the sequencing of the present invention contained a small proportion of duplicates. Gene predictions were estimated using the Cufflinks method (Trapnell, C., et al. Nat. Biotechnol. 28, 511-515, 2010) and expression levels were calculated using HT Seq software (version 0.6.0, http://www-huber.embl.de/users/anders/HTSeq/). This method uses single reads for estimating gene expression and eliminates multiple mapped reads. The differential expression analysis between conditions was evaluated using the DESeq2 method (Love, M.I. et al., Genome Biol 15, 2014, version 3.4). Finally, differentially expressed genes with an FDR-adjusted p-value (Benjamini, Y., et al. J Roy Stat Soc B 57, 1995) <0.05 and a fold change of at least 1.5 were selected. DEG analysis between S and A was performed using statistical packages designed by Python and R. By using the DESseq2 algorithm (Anders, S. et al., Genome Biol 11, 2010), which applies a differential negative binomial distribution for statistical significance (Love, M.I. et al., Genome Biol 15, 2014, version 3.4), differentially expressed genes and isoforms were identified. Differentially expressed genes or isoforms were considered to be those genes or isoforms with an FC value less than -1.5 or greater than 1.5, and with an FDR-adjusted p-value (*P_{adjus}t*) ≤0.05 (Benjamini, Y., et al., J Roy Stat Soc B 57, 1995) to prevent the identification of false positives in the differential expression data.

### Functional enrichment analysis and network analysis of the set of genes

Differentially expressed sets were processed using ClusterProfiler (Yu, G., et al. Omi. a J. Integr. Biol. 16, 284-287 (2012), a bioconductor package, to look for biological processes involved in plaque instability. This tool studies genes in specific databases (i.e., Gene Ontology - GO, Kyoto Encyclopedia of Genes and Genomes - KEGG, DRUG, etc.) to evaluate biological annotations that come up as being over-represented with respect to the whole genome. Gene networks were generated using GeneMANIA software (http://www.genemania.org/). GeneMANIA uses functional interconnections between genes from published data to generate an overall view of gene interactions. The MCODE tool (http.://apps.cytoscape.org/apps/mcode) was used to identify highly interconnected clusters in a network.

### Real-time quantitative PCR

SYBRgreen technology was used to analyze the gene expression of CXCL9, CXCL10, CD5L, and BMP2 as a quality control and to control the purity of the SMC cultures. PrimeTime assays for qPCR (IDT, Leuven, Belgium) were used, and GAPDH was used as constitutives for normalization. Gene expression levels were detected with ABI7500Fast equipment (Life Technologies, Carlsbad, CA, USA). The analysis was performed with the ΔCt method and expressed as 2(-ΔCt). The significance was calculated as previously described using GraphPad Prism 5 software.

### Digital PCR in the Fluidigm Biomark platform

Assays (pre-designed PrimeTime primers) for 120 genes previously identified by means of RNAseq were acquired from IDT. Four genes were used as housekeeping genes for normalization (G3BP2, MKLN1, EML3, and ADCK5). The RNA was converted into cDNA using the AffinityScript Multiple Temperature cDNA Synthesis kit (Agilent, Technologies). The analysis of the expression was performed with Fluidigm Biomark 96.96 arrays (Fluidigm Corp.) and PrimeTime assays in 189 samples in duplicate. The expression levels of the housekeeping genes have been used as an endogenous control to normalize the samples. The relative expression levels have been calculated using the deltadeltaCt method. The statistical significance of the expression levels between S and A has been analyzed with GraphPad Prism 5 software using the non-parametric Mann-Whitney U-test. All the PrimeTime assays showed amplification efficiencies close to 100%.

### Example 1: Experimental approach to distinguish SMCs (smooth muscle cells) isolated from symptomatic and asymptomatic carotid plaques by means of RNAseq technology

The study was designed with maximum power to detect DEGs (differentially expressed genes) and DEIs (differentially expressed isoforms), as well as to perform complementary functional enrichment and network analyses. The two main factors to be taken into account in the transcriptomic studies by means of RNAseq are the number of repetitions and sequencing depth, such that RNA sequencing with a depth that is sufficient to reach a reliable differential expression detection power to obtain about 60 million reads per sample was performed. Sequencing at this depth allows identifying differential expression with maximum power in 5 repetitions, even in those genes with low expression. It furthermore allows reducing variability in the RNAseq analysis when a specific cell type (i.e., SMCs) is studied compared with the study of the entire atheromatous plaque, which is heterogeneous and made up of a variable proportion of endothelial cells, SMCs, and macrophages. Accordingly, 14 (7 symptomatic and 7 asymptomatic) patients proposed for carotid endarterectomy by the committee of medical experts of Hospital Universitario of Basurto, who evaluated the clinical data of the patients (table 4), were selected to study the role of SMCs in the development of unstable atherosclerotic plaques. The radiology department performed an MRI and cervical Doppler ultrasound in all the patients in the hospital. Patients with >70% stenosis and diagnosed with a cerebrovascular event and patients with >80% stenosis and with no events were included in the study. Other required parameters besides being symptomatic (S) and asymptomatic (A) for selecting eligible patients to be included in this study were defined as: (1) age (70 years±12); (2) no other medical conditions; and (3) no prior contralateral endarterectomy. During the inclusion period, patients were excluded from the study for having at least one of the following problems: (1) patients who showed a sign of dementia according to the mini-mental state examination (MMSE) test; (2) patients with psychological impairments due to drugs or brain surgery; (3) patients who presented medical or surgical complications that may have altered their intellectual capacity and/or the neuroimaging test; and (4) patients who were unable to perform the neuropsychological test due to a communication difficulty (i.e., vision impairment).

**Table 4: Clinical data of the patients included in the study.**

| Characteristics of patients | All | Symptomatic | Asymptomatic |
|---|---|---|---|
| Number, n | 14 | 7 | 7 |
| Years | 68 | 68±8 | 68±8 |
| Sex F/M, n | 11/3 | 4/3 | 7/0 |
| Treatment with statins | 14 | 7 | 7 |

| Risk factors (%) | | | |
|---|---|---|---|
| Diabetes mellitus | 100 | 3 (43%) | 4 (57%) |
| Dyslipidemia | 100 | 7 (100%) | 7 (100%) |
| High blood pressure | 100 | 7 (100%) | 7 (100%) |
| Tobacco | 100 | 4 (57%) | 3 (43%) |

### Example 2: The SMCs isolated from carotid atherosclerotic plaque express typical biomarkers of SMC

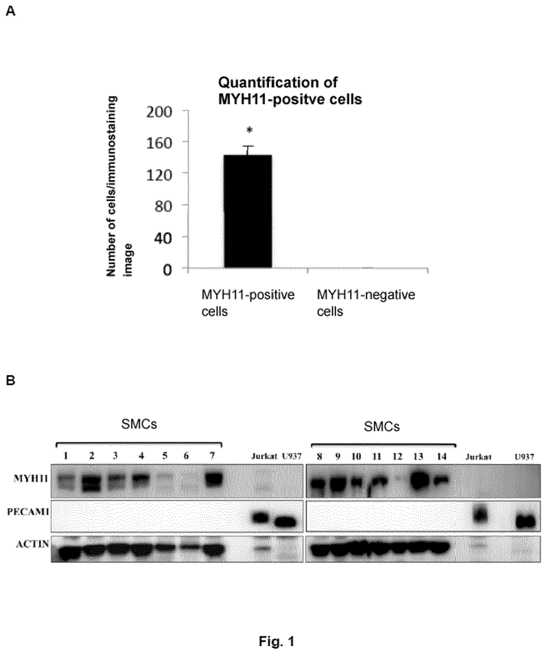

It is known in the state of the art that the isolation and culture of primary vascular SMCs is considered an acceptable strategy for studying the role of SMCs in atherosclerosis, which can be grouped as isolation by explant or isolation based on enzymatic digestion. The second option generated a larger number of isolated cells. Particularly, to isolate the SMCs from the atheromatous carotid plaque a previously described enzymatic method was used, with some modifications (Bonano et al., 2000. Cytometry 39, 158-165). The atheroma plaque tissue samples were sectioned into 2.3 mm sized fragments and then digested with collagenase I. First digestion was performed for 3 hours with 300 U/ml of collagenase I. Then the digested tissue was subjected to a second digestion for 16 hours with 200 U/ml of collagenase I. The digested tissue is filtered with 100 µm filters to remove the undigested material. The cells are cultured in a medium specific for M231 supplemented with growth factors. To determine the efficacy of isolation and the purity of the primary SMC culture, two quality control tests were performed. First, markers specific for smooth muscle cells were detected by means of immunostaining using antibodies specific for SMCs (i.e., α-MYH11) (Ross, R. J. Cell Biol. 50, 172-186 (1971) and endothelial cells (PECAM1- as a negative control), which allowed analyzing the homogeneity of the primary SMC cultures. It was also detected that in the SMC cultures there was no macrophage contamination by means of identifying specific macrophage markers (i.e., CXCL9, CXCL10, and CD5L). In summary, the cells were seeded in 24-well plates with glass slides and the cells were stained with α-MYH11 antibodies. Image acquisition was performed using a Leica TCS STED CW SP8 microscope. All the SMC cultures used in the study were 100% MYH11-positive. Figure 1A shows the quantification of the MYH11-positive cells. Then, the intracellular expression of specific SMC proteins (MYH1 and PECAM1) was analyzed. The Western blot analysis showed that the isolated SMCs expressed the specific tested SMC biomarkers and that they were negative for PECAM1 expression. Figure 1B shows an illustration of the detection by means of Western blot with α-PECAM1 and α-MHY11 antibodies in cell lysates extracted from SMC cultures. Cell cultures in passage 0-2 were used for quality control analysis.

### Example 3: Analysis of DEG and DEI with transcriptomic data

To prevent possible changes in transcription due to the number of passages, the RNA used for the RNAseq analysis was extracted in all cases from smooth muscle cells in passage 0. RNA integrity (RIN) was evaluated to assure good RNA quality. All the RNA samples showed an RIN value between 9 and 10, which classified them as samples suitable for RNAseq analysis. A comparative transcriptomic analysis between the two groups, A and S, was performed by means of applying RNAseq technology to understand the role of SMCs in the symptomatology of carotid atherosclerosis. The number of repetitions per group (n=7) was decided on according to the maximum availability of tissue obtained in a specific period taking into account the minimum repetitions recommended for studies of this type. The 14 libraries generated were sequenced in the Illumina Hiseq2500 platform. The data obtained with RNA sequencing was converted into quantitative levels of gene expression with the aid of the identification of differentially expressed genes (DEGs) and differentially expressed isoforms (DEIs) by comparing A and S. Between 85 and 87% of mapped reads were obtained from the total reads generated (Table 5), which was within the expected range of mapped reads in the human genome (70-90%) Risso, D. et al., BMC Bioinformatics 12, (2011).

**Table 5. RNAseq mapping data summary. HQ, high quality.**

| Sample ID | Total reads | % of HQ reads | % of properly paired reads | % of spliced reads |
|---|---|---|---|---|
| 1 | 61449044 | 86.11 | 72.15 | 26.38 |
| 2 | 76876864 | 86.51 | 71.27 | 25.33 |
| 3 | 67990002 | 85.99 | 70.8 | 25.14 |
| 4 | 67892558 | 86.08 | 70.74 | 26.91 |
| 5 | 71976326 | 86.78 | 72.31 | 26.78 |
| 6 | 74497186 | 82.21 | 72.82 | 23.61 |
| 7 | 72479266 | 87. 66 | 71.85 | 27.01 |
| 8 | 70654274 | 84.75 | 72.09 | 24.79 |
| 9 | 73679062 | 87.23 | 71.45 | 27.43 |
| 10 | 70845658 | 86.19 | 71.21 | 25.85 |
| 11 | 72278906 | 86.59 | 72.24 | 25.42 |
| 12 | 72592062 | 86.26 | 70. 91 | 27.36 |
| 13 | 80682878 | 85.84 | 73.04 | 26.56 |
| 14 | 72040720 | 86.47 | 70. 94 | 26.49 |

The RNAseq data had to be normalized to eliminate any statistical deviation that could alter subsequent analysis. The main important deviations found were gene length, library size, and GC content. To prevent such deviations, normalization was performed using the proposed DESeq2 method (Hansen, K.D., et al. Biostatistics 13, 204-216 2012). Primary component analysis (PCA) was performed in the gene expression profiles using the previously published method (Stacklies, W., et al., Bioinforma. 23, 1164-1167 (2007) to identify possible atypical values. The PCA obtains 2 atypical differentiated values (A7 and S1), which would increase variability and decrease the power of the present statistical data, and therefore they were eliminated from the subsequent analysis. Dispersion fit illustrates how much the variance deviates from the mean and this was used to shrink the final estimates from the gene-wise estimates towards the fitted estimates, which improves gene expression analysis. In differential expression analysis, 93 genes were identified in DEG analysis. A complete list of the 93 genes can be found in Table 5, which shows FC values ≥1.5 with a p-value ≤0.05 (67 with adjusted p-value (*P_{adjust}*) ≤0.05 (Benjamini, Y. J Roy Stat Soc B 57, 1995)). Among the genes included in Table 4, the genes are associated with: cell *growth and senescence, cell metabolism, retinol metabolism; vascular disease, autophagy, immune system, development*/*muscle contraction.*

**Table 6. Differential gene expression between S (symptomatic) and A (asymptomatic). The fold change (FC) value is obtained by means of the DeSeq2 method and the adjusted p-value (P_{adjust}) was calculated using the false discovery rate (FDR) method. A significant P_{adjust} value was considered P_{adjust}≤0.05. *Genes showing a propensity for significance but without reaching it (P_{adjust} ≤0.09).**

| Gene ID | Symbol | FC | *P_{adjust}* | FC (Fluidigm) | *P* (Fluidigm) |
|---|---|---|---|---|---|
| ENSG00000165507 | C10orf10 | 1.94 | 0.0006 | Undif. FC | not significant |
| ENSG00000152049 | KCNE4 | 1.87 | 0.003 | Undif. FC | not significant |
| **ENSG00000117069** | **ST6GALNAC5** | **1.83** | **0.009** | **3.922** | **0.041** |
| **ENSG00000135424** | **ITGA7** | **1.81** | **0.005** | **2.39** | **0.05** |
| ENSG00000128285 | MCHR1 | 1.76 | 0.02 | n.a. | n.a. |
| ENSG00000174564 | IL20RB | 1.75 | 0.02 | Undif. FC | not significant |
| ENSG00000163815 | CLEC3B | 1.75 | 0.02 | Undif. FC | not significant |
| ENSG00000075643 | MOCOS | 1.75 | 0.001 | 1.31 | 0.05 |
| **ENSG00000074410** | **CA12** | **1.72** | **0.0006** | **2.7** | **0.0048** |
| ENSG00000174807 | CD248 | 1.71 | 0.03 | Undif. FC | not significant |
| ENSG00000133454 | MY018B | 1.70 | 0.04 | Undif. FC | not significant |
| ENSG00000179542 | SLITRK4 | 1.65 | 0.06^{*} | Undif. FC | not significant |
| ENSG00000161570 | CCL5 | 1.65 | 0.06^{*} | Undif. FC | not significant |
| ENSG00000148948 | LRRC4C | 1.65 | 0.06^{*} | n.a. | n.a. |
| ENSG00000117289 | TXNIP | 1.64 | 0.04 | n.a. | n.a. |
| ENSG00000123689 | GOS2 | 1.63 | 0.05 | n.a. | n.a. |
| ENSG00000162706 | CADM3 | 1.63 | 0.07* | Undif. FC | not significant |
| ENSG00000120088 | CRHR1 | 1.62 | 0.07* | n.a. | n.a. |
| ENSG00000184557 | SOCS3 | 1.61 | 0.03 | Undif. FC | not significant |
| **ENSG00000176438** | **SYNE 3** | **1.60** | **0.05** | **2.9** | **0.034** |
| ENSG00000161958 | FGF11 | 1.60 | 0.04 | n.a. | n.a. |
| ENSG00000234745 | HLA-B | 1.59 | 0.06* | n.a. | n.a. |
| ENSG00000164236 | ANKRD33B | 1.59 | 0.09* | n.a. | n.a. |
| ENSG00000170624 | SGCD | 1.57 | 0.08* | Undif. FC | not significant |
| ENSG00000259649 | RP11-351M8.1 | 1.57 | 0.09* | n.a. | n.a. |
| ENSG00000172201 | ID4 | 1.55 | 0.09* | Undif. FC | not significant |
| ENSG00000119938 | PPP1R3C | 1.54 | 0.09* | Undif. FC | not significant |
| **ENSG00000114268** | **PFKFB4** | **1.54** | **0.04** | **2.86** | **0.029** |
| ENSG00000110076 | NRXN2 | 1.53 | 0.05 | Undif. FC | not significant |
| **ENSG00000136378** | **ADAMTS7** | **1.52** | **0.02** | **1.23** | **0.08** |
| **ENSG00000152256** | **PDK1** | **1.52** | **0.03** | **2.59** | **0.029** |
| **ENSG00000112837** | **TBX18** | **1.51** | **0.09*** | **3.7** | **0.03** |
| **ENSG00000162733** | **DDR2** | **1.50** | **0.02** | **3.817** | **0.05** |
| ENSG00000179348 | GATA2 | -1.51 | 0.09* | n.a. | n.a. |
| ENSG00000120693 | SMAD9 | -1.52 | 0.05 | Undif. FC | not significant |
| **ENSG00000164736** | **SOX17** | **-1.52** | **0.09*** | **-4.43** | **0.039** |
| ENSG00000035664 | DAPK2 | -1.53 | 0.09* | n.a. | n.a. |
| **ENSG00000144730** | **IL17RD** | **-1.54** | **0.01** | **-1.545** | **0.04** |
| ENSG00000133216 | EPHB2 | -1.55 | 0.03 | Undif. FC | not significant |
| ENSG00000166960 | CCDC178 | -1.55 | 0.09* | n.a. | n.a. |
| ENSG00000120279 | MYCT1 | -1.57 | 0.05 | n.a. | n.a. |
| ENSG00000121039 | RDH10 | -1.57 | 0.06* | Undif. FC | not significant |
| ENSG00000183578 | TNFAIP8L3 | -1.58 | 0.09* | Undif. FC | not significant |
| ENSG00000145681 | HAPLN1 | -1.58 | 0.09* | Undif. FC | not significant |
| ENSG00000158125 | XDH | -1.59 | 0.09* | n.a | n.a |
| ENSG00000139174 | PRICKLE1 | -1.59 | 0.08* | n.a | n.a |
| ENSG00000182368 | FAM27A | -1.59 | 0.09* | n.a | n.a |
| **ENSG00000118137** | **APOA1** | **-1.60** | **0.07*** | **-4.33** | **0.04** |
| ENSG00000169684 | CHRNA5 | -1.60 | 0.09* | Undif. FC | not significant |
| ENSG00000167644 | C19orf33 | -1.61 | 0.06^{*} | n.a. | n.a. |
| ENSG00000116106 | EPHA4 | -1.61 | 0.05 | Undif. FC | not significant |
| **ENSG00000125968** | **ID1** | **-1.61** | **0.04** | **-9.19** | **0.03** |
| **ENSG00000123572** | **NRK** | **-1.61** | **0.09*** | **-1.48** | **0.026** |
| ENSG00000121361 | KCNJ8 | -1.63 | 0.07* | n.a. | n.a. |
| ENSG00000091129 | NRCAM | -1.63 | 0.07* | Undif. FC | not significant |
| ENSG00000175746 | C15orf54 | -1.64 | 0.05 | n.a. | n.a. |
| ENSG00000172602 | RND1 | -1.64 | 0.04 | n.a. | n.a. |
| ENSG00000095752 | IL11 | -1.64 | 0.07* | n.a. | n.a. |
| ENSG00000175866 | BAIAP2 | -1.65 | 0.01 | n.a. | n.a. |
| **ENSG00000127329** | **PTPRB** | **-1.65** | **0.06^{*}** | **-1.19** | **0.08** |
| ENSG00000144366 | GULP1 | -1.66 | 0.005 | Undif. FC | not significant |
| ENSG00000143140 | GJA5 | -1.66 | 0.04 | n.a. | n.a. |
| ENSG00000176399 | DMRTA1 | -1.68 | 0.05 | n.a. | n.a. |
| ENSG00000168497 | SDPR | -1.68 | 0.02 | Undif. FC | not significant |
| ENSG00000010319 | SEMA3G | -1.69 | 0.02 | n.a. | n.a. |
| ENSG00000254656 | RTL1 | -1.69 | 0.03 | n.a. | n.a. |
| ENSG00000178445 | GLDC | -1.69 | 0.02 | n.a. | n.a. |
| **ENSG00000162496** | **DHRS3** | **-1.70** | **0.04** | **-1.67** | **0.018** |
| **ENSG00000102554** | **KLF5** | **-1.70** | **0.003** | **-1.15** | **0.05** |
| **ENSG00000172159** | **FRMD3** | **-1.70** | **0.04** | **-1.6** | **0.0071** |
| ENSG00000164283 | ESM1 | -1.71 | 0.03 | Undif. FC | not significant |
| ENSG00000235109 | ZSCAN31 | -1.72 | 0.01 | Undif. FC | not significant |
| ENSG00000132321 | IQCA1 | -1.72 | 0.02 | n.a. | n.a. |
| ENSG00000008394 | MGST1 | -1.72 | 0.002 | Undif. FC | not significant |
| **ENSG00000164530** | **PI16** | **-1.73** | **0.01** | **-8.92** | **0.00218** |
| ENSG00000150722 | PPP1R1C | -1.73 | 0.03 | n.a. | n.a. |
| ENSG00000116741 | RGS2 | -1.74 | 0.02 | Undif. FC | not significant |
| ENSG00000180660 | MAB21L1 | -1.76 | 0.02 | n.a. | n.a. |
| ENSG00000155011 | DKK2 | -1.77 | 0.01 | Undif. FC | not significant |
| ENSG00000126950 | TMEM35 | -1.81 | 0.02 | Undif. FC | not significant |
| ENSG00000179104 | TMTC2 | -1.81 | 0.004 | Undif. FC | not significant |
| **ENSG00000118777** | **ABCG2** | **-1.85** | **0.01** | **-1.9** | **0.03** |
| ENSG00000125848 | FLRT3 | -1.87 | 0.007 | n.a. | n.a. |
| ENSG00000108375 | RNF43 | -1.90 | 0.003 | n.a. | n.a. |
| **ENSG00000125845** | **BMP2** | **-1.90** | **0.005** | **Undif. FC** | **not significant** |
| **ENSG00000138759** | **FRAS1** | **-1.90** | **0.005** | **-10.38** | **0.018** |
| ENSG00000108556 | CHRNE | -1.91 | 0.005 | n.a. | n.a. |
| ENSG00000163273 | NPPC | -1.93 | 0.003 | Undif. FC | not significant |
| ENSG00000162595 | DIRAS3 | -1.93 | 0.003 | Undif. FC | not significant |
| ENSG00000109511 | ANXA10 | -1.93 | 0.003 | Undif. FC | not significant |
| ENSG00000154645 | CHODL | -2.16 | 0.0003 | Undif. FC | not significant |
| ENSG00000138772 | ANXA3 | -2.23 | 0.0001 | Undif. FC | not significant |
| ENSG00000087494 | PTHLH | -2.50 | 1.04E-07 | Undif. FC | not significant |

**Table 7. Differential gene expression between S (symptomatic) and A (asymptomatic). The fold change (FC) value is obtained by means of the DeSeq2 method and the adjusted p-value (P_{adjust}) was calculated using the false discovery rate (FDR) method. A significant P_{adjust} value was considered P_{adjust}≤0.05. *Genes showing a propensity for the significance but without reaching it (P_{adjust} ≤0.09).**

| Gene ID | Symbol | FC (RNAseq) | *P*_{adj} (RNAseq) | FC (Fluidigm) | *P* (Fluidigm) |
|---|---|---|---|---|---|
| ENSG00000184014 | DENND5A | 1.21 | 0.01 | 1.28505305 | 0.09 |
| ENSG00000165948 | IFI27L1 | -1.3 | 0.04 | -2.360236804 | 0.05 |
| ENSG00000101577 | LPIN2 | 1.21 | 0.01 | 1.24344667 | 0.06 |
| ENSG00000165406 | MARCH8 | 1.3 | 0.04 | 1.3127485 | 0.0003 |
| ENSG00000156103 | MMP16 | -1.34 | 0.05 | -2.077236984 | 0.021 |
| ENSG00000116711 | PLA2G4A | -1.4 | 0.05 | -1.293069257 | 0.0218 |
| ENSG00000122861 | PLAU | 1.4 | 0.05 | 1.975451153 | 0.09 |
| ENSG00000132563 | REEP2 | -1.4 | 0.004 | -2.406583811 | 0.024 |
| ENSG00000073060 | SCARB1 | 1.24 | 0.01 | 1.404153243 | 0.05 |
| ENSG00000155850 | SLC26A2 | -1.35 | 0.01 | -1.476862205 | 0.05 |
| ENSG00000059804 | SLC2A3 | 1.32 | 0.05 | 1.568040574 | 0.03 |

The exact quantification of isoforms continues to be a challenge due to the high degree of overlap between transcripts. Variability in precision of the estimate of the isoform expression through the samples was biased using conventional differential expression packages. For this reason, in this case the Bayesian Cufflink approach was used, which estimates the abundance of transcripts based on how many reads each transcript supports. DEI analysis identified 143 differentially expressed isoforms between S and A. Table 6 illustrates isoforms with an FC value less than -1.5 or greater than 1.5 and with a *P_{adjust}* ≤ 0.05.

**Table 8: Analysis of the differential isoform expression between symptomatic and asymptomatic patients using the Cufflinks method. The FC value was calculated using the DeSeq1 method and P_{adj} was calculated by applying FDR. The significant P_{adj} value considered was P_{adjust} ≤0.05.**

| ID isoforms | Gene ID | Symbol | FC | *P* ᵥₐₗᵤₑ | *P_{adjust}* |
|---|---|---|---|---|---|
| ENST00000358321 | ENSG00000099994 | SUSD2 | 11.61 | 5.00E-05 | 0.01 |
| ENST00000260227 | ENSG00000137673 | MMP7 | 9.25 | 5.00E-05 | 0.01 |
| ENST00000506608 | ENSG00000185149 | NPY2R | 8.39 | 0.0002 | 0.04 |
| ENST00000264613 | ENSG00000047457 | CP | 7.90 | 0.0002 | 0.04 |
| ENST00000374975 | ENSG00000198502 | HLA-DRB5 | 5.72 | 5.00E-05 | 0.01 |
| ENST00000477717 | ENSG00000117069 | ST6GALNAC5 | 4.84 | 5.00E-05 | 0.01 |
| ENST00000075322 | ENSG00000136960 | ENPP2 | 4.59 | 5.00E-05 | 0.01 |
| ENST00000296130 | ENSG00000163815 | CLEC3B | 4.37 | 5.00E-05 | 0.01 |
| ENST00000394887 | ENSG00000196616 | ADH1B | 4.07 | 5.00E-05 | 0.01 |
| ENST00000259486 | ENSG00000136960 | ENPP2 | 3.88 | 5.00E-05 | 0.01 |
| ENST00000013222 | ENSG00000241644 | INMT | 3.84 | 5.00E-05 | 0.01 |
| ENST00000393078 | ENSG00000196136 | SERPINA3 | 3.57 | 5.00E-05 | 0.01 |
| ENST00000368125 | ENSG00000162706 | CADM3 | 3.53 | 0.00015 | 0.03 |
| ENST00000296506 | ENSG00000164106 | SCRG1 | 3.43 | 5.00E-05 | 0.01 |
| ENST00000256861 | ENSG00000123243 | ITIH5 | 3.42 | 0.00015 | 0.03 |
| ENST00000294728 | ENSG00000162692 | VCAM1 | 3.38 | 5.00E-05 | 0.01 |
| ENST00000274938 | ENSG00000146197 | SCUBE3 | 3.30 | 5.00E-05 | 0.01 |
| ENST00000167586 | ENSG00000186847 | KRT14 | 3.06 | 5.00E-05 | 0.01 |
| ENST00000374737 | ENSG00000155659 | VSIG4 | 3 | 0.00015 | 0.03 |
| ENST00000372330 | ENSG00000100985 | MMP9 | 2.82 | 5.00E-05 | 0.01 |
| ENST00000447544 | ENSG00000135218 | CD36 | 2.65 | 5.00E-05 | 0.01 |
| ENST00000298295 | ENSG00000165507 | C10orf10 | 2.50 | 5.00E-05 | 0.01 |
| ENST00000604125 | ENSG00000152049 | KCNE4 | 2.48 | 5.00E-05 | 0.01 |
| ENST00000542220 | ENSG00000159403 | C1R | 2.42 | 5.00E-05 | 0.01 |
| ENST00000553804 | ENSG00000135424 | ITGA7 | 2.38 | 5.00E-05 | 0.01 |
| ENST00000367662 | ENSG00000116183 | PAPPA2 | 2.34 | 0.00015 | 0.03 |
| ENST00000006053 | ENSG00000006210 | CX3CL1 | 2.29 | 5.00E-05 | 0.01 |
| ENST00000361673 | ENSG00000198796 | ALPK2 | 2.20 | 5.00E-05 | 0.01 |
| ENST00000437043 | ENSG00000164308 | ERAP2 | 2.18 | 0.0001 | 0.02 |
| ENST00000339732 | ENSG00000131386 | GALNT15 | 2.15 | 0.0002 | 0.04 |
| ENST00000284322 | ENSG00000154175 | ABI3BP | 2.12 | 5.00E-05 | 0.01 |
| ENST00000261799 | ENSG00000113721 | PDGFRB | 2.10 | 5.00E-05 | 0.01 |
| ENST00000369317 | ENSG00000117289 | TXNIP | 2.07 | 5.00E-05 | 0.01 |
| ENST00000435422 | ENSG00000170624 | SGCD | 2.06 | 5.00E-05 | 0.01 |
| ENST00000345517 | ENSG00000163017 | ACTG2 | 2 | 0.00015 | 0.03 |
| ENST00000257435 | ENSG00000134986 | NREP | 1.99 | 5.00E-05 | 0.01 |
| ENST00000429713 | ENSG00000172403 | SYNPO2 | 1.99 | 5.00E-05 | 0.01 |
| ENST00000412585 | ENSG00000234745 | HLA-B | 1.98 | 5.00E-05 | 0.01 |
| ENST00000307365 | ENSG00000168209 | DDIT4 | 1.96 | 5.00E-05 | 0.01 |
| ENST00000261326 | ENSG00000075643 | MOCOS | 1.95 | 5.00E-05 | 0.01 |
| ENST00000178638 | ENSG00000074410 | CA12 | 1.94 | 5.00E-05 | 0.01 |
| ENST00000254722 | ENSG00000132386 | SERPINF1 | 1.93 | 5.00E-05 | 0.01 |
| ENST00000335174 | ENSG00000186352 | ANKRD37 | 1.90 | 0.0002 | 0.04 |
| ENST00000378700 | ENSG00000172201 | ID4 | 1.89 | 5.00E-05 | 0.01 |
| ENST00000294507 | ENSG00000162511 | LAPTM5 | 1.89 | 5.00E-05 | 0.01 |
| ENST00000540010 | ENSG00000056558 | TRAF1 | 1.89 | 5.00E-05 | 0.01 |
| ENST00000330871 | ENSG00000184557 | SOCS3 | 1.88 | 5.00E-05 | 0.01 |
| ENST00000344366 | ENSG00000074410 | CA12 | 1.87 | 5.00E-05 | 0.01 |
| ENST00000238994 | ENSG00000119938 | PPP1R3C | 1.86 | 5.00E-05 | 0.01 |
| ENST00000217233 | ENSG00000101255 | TRIB3 | 1.84 | 5.00E-05 | 0.01 |
| ENST00000327299 | ENSG00000162433 | AK4 | 1.83 | 0.0002 | 0.04 |
| ENST00000328916 | ENSG00000182326 | C1S | 1.79 | 0.0001 | 0.02 |
| ENST00000323076 | ENSG00000136167 | LCP1 | 1.76 | 5.00E-05 | 0.01 |
| ENST00000157600 | ENSG00000071282 | LMCD1 | 1.75 | 0.0001 | 0.02 |
| ENST00000297848 | ENSG00000187955 | COL14A1 | 1.73 | 5.00E-05 | 0.01 |
| ENST00000369663 | ENSG00000112837 | TBX18 | 1.73 | 5.00E-05 | 0.01 |
| ENST00000367288 | ENSG00000163431 | LMOD1 | 1.72 | 5.00E-05 | 0.01 |
| ENST00000338981 | ENSG00000114374 | USP9Y | 1.70 | 5.00E-05 | 0.01 |
| ENST00000566620 | ENSG00000140945 | CDH13 | 1.68 | 5.00E-05 | 0.01 |
| ENST00000394308 | ENSG00000070669 | ASNS | 1.65 | 0.0001 | 0.02 |
| ENST00000402744 | ENSG00000109472 | CPE | 1.65 | 5.00E-05 | 0.01 |
| ENST00000367922 | ENSG00000162733 | DDR2 | 1.64 | 5.00E-05 | 0.01 |
| ENST00000220244 | ENSG00000103888 | KIAA1199 | 1.62 | 0.0002 | 0.04 |
| ENST00000380250 | ENSG00000073910 | FRY | 1.58 | 0.00015 | 0.03 |
| ENST00000409652 | ENSG00000221963 | APOL6 | 1.56 | 0.00015 | 0.03 |
| ENST00000396680 | ENSG00000128272 | ATF4 | 1.55 | 0.00015 | 0.03 |
| ENST00000372764 | ENSG00000122861 | PLAU | 1.55 | 0.0001 | 0.02 |
| ENST00000375377 | ENSG00000165757 | KIAA1462 | 1.54 | 0.00015 | 0.03 |
| ENST00000263734 | ENSG00000116016 | EPAS1 | 1.53 | 5.00E-05 | 0.01 |
| ENST00000295992 | ENSG00000163710 | PCOLCE2 | 1.53 | 5.00E-05 | 0.01 |
| ENST00000376588 | ENSG00000135069 | PSAT1 | 1.53 | 0.0002 | 0.04 |
| ENST00000274276 | ENSG00000145623 | OSMR | 1.50 | 0.0002 | 0.04 |
| ENST00000358432 | ENSG00000142627 | EPHA2 | -1.50 | 5.00E-05 | 0.01 |
| ENST00000260630 | ENSG00000138061 | CYP1B1 | -1.53 | 0.00015 | 0.03 |
| ENST00000229416 | ENSG00000001084 | GCLC | -1.56 | 0.0002 | 0.04 |
| ENST00000367466 | ENSG00000116711 | PLA2G4A | -1.56 | 0.0001 | 0.02 |
| ENST00000005257 | ENSG00000006451 | RALA | -1.56 | 0.0001 | 0.02 |
| ENST00000245185 | ENSG00000125148 | MT2A | -1.57 | 0.0001 | 0.02 |
| ENST00000361441 | ENSG00000244509 | APOBEC3C | -1.58 | 0.0001 | 0.02 |
| ENST00000381434 | ENSG00000137033 | IL33 | -1.62 | 0.0001 | 0.02 |
| ENST00000272542 | ENSG00000144136 | SLC20A1 | -1.63 | 5.00E-05 | 0.01 |
| ENST00000371697 | ENSG00000148677 | ANKRD1 | -1.64 | 0.00015 | 0.03 |
| ENST00000248598 | ENSG00000127951 | FGL2 | -1.67 | 5.00E-05 | 0.01 |
| ENST00000315711 | ENSG00000091972 | CD200 | -1.68 | 0.0002 | 0.04 |
| ENST00000222543 | ENSG00000105825 | TFPI2 | -1.69 | 5.00E-05 | 0.01 |
| ENST00000237305 | ENSG00000118515 | SGK1 | -1.72 | 5.00E-05 | 0.01 |
| ENST00000367468 | ENSG00000073756 | PTGS2 | -1.77 | 5.00E-05 | 0.01 |
| ENST00000258888 | ENSG00000136383 | ALPK3 | -1.78 | 5.00E-05 | 0.01 |
| ENST00000426693 | ENSG00000164619 | BMPER | -1.81 | 0.0002 | 0.04 |
| ENST00000379375 | ENSG00000078401 | EDN1 | -1.86 | 5.00E-05 | 0.01 |
| ENST00000276925 | ENSG00000147883 | CDKN2B | -1.91 | 5.00E-05 | 0.01 |
| ENST00000377861 | ENSG00000184226 | PCDH9 | -1.91 | 5.00E-05 | 0.01 |
| ENST00000265080 | ENSG00000113319 | RASGRF2 | -1.91 | 5.00E-05 | 0.01 |
| ENST00000377103 | ENSG00000178726 | THBD | -1.93 | 5.00E-05 | 0.01 |
| ENST00000376112 | ENSG00000125968 | ID1 | -1.99 | 5.00E-05 | 0.01 |
| ENST00000252593 | ENSG00000130303 | BST2 | -2.03 | 5.00E-05 | 0.01 |
| ENST00000396210 | ENSG00000008394 | MGST1 | -2.04 | 5.00E-05 | 0.01 |
| ENST00000330688 | ENSG00000077092 | RARB | -2.04 | 5.00E-05 | 0.01 |
| ENST00000310613 | ENSG00000173597 | SULT1B1 | -2.09 | 0.0001 | 0.02 |
| ENST00000327536 | ENSG00000183578 | TNFAIP8L3 | -2.09 | 5.00E-05 | 0.01 |
| ENST00000274341 | ENSG00000145681 | HAPLN1 | -2.10 | 5.00E-05 | 0.01 |
| ENST00000304141 | ENSG00000168497 | SDPR | -2.10 | 5.00E-05 | 0.01 |
| ENST00000262259 | ENSG00000105497 | ZNF175 | -2.15 | 5.00E-05 | 0.01 |
| ENST00000306773 | ENSG00000171246 | NPTX1 | -2.21 | 5.00E-05 | 0.01 |
| ENST00000222482 | ENSG00000128510 | CPA4 | -2.27 | 5.00E-05 | 0.01 |
| ENST00000377474 | ENSG00000178695 | KCTD12 | -2.35 | 5.00E-05 | 0.01 |
| ENST00000321196 | ENSG00000179104 | TMTC2 | -2.35 | 5.00E-05 | 0.01 |
| ENST00000315274 | ENSG00000196611 | MMP1 | -2.40 | 0.00015 | 0.03 |
| ENST00000444842 | ENSG00000082482 | KCNK2 | -2.43 | 5.00E-05 | 0.01 |
| ENST00000286614 | ENSG00000156103 | MMP16 | -2.43 | 5.00E-05 | 0.01 |
| ENST00000376468 | ENSG00000120937 | NPPB | -2.43 | 5.00E-05 | 0.01 |
| ENST00000235382 | ENSG00000116741 | RGS2 | -2.45 | 5.00E-05 | 0.01 |
| ENST00000546561 | ENSG00000127324 | TSPAN8 | -2.54 | 5.00E-05 | 0.01 |
| ENST00000541194 | ENSG00000128567 | PODXL | -2.59 | 5.00E-05 | 0.01 |
| ENST00000247829 | ENSG00000127324 | TSPAN8 | -2.59 | 5.00E-05 | 0.01 |
| ENST00000232892 | ENSG00000114771 | AADAC | -2.69 | 5.00E-05 | 0.01 |
| ENST00000297785 | ENSG00000165092 | ALDH1A1 | -2.72 | 5.00E-05 | 0.01 |
| ENST00000591504 | ENSG00000166510 | CCDC68 | -2.74 | 5.00E-05 | 0.01 |
| ENST00000307407 | ENSG00000169429 | IL8 | -2.82 | 5.00E-05 | 0.01 |
| ENST00000284136 | ENSG00000153993 | SEMA3D | -2.85 | 5.00E-05 | 0.01 |
| ENST00000537928 | ENSG00000128567 | PODXL | -2.86 | 0.0002 | 0.04 |
| ENST00000376223 | ENSG00000162496 | DHRS3 | -2.87 | 5.00E-05 | 0.01 |
| ENST00000304195 | ENSG00000172159 | FRMD3 | -3 | 0.00015 | 0.03 |
| ENST00000359299 | ENSG00000109511 | ANXA10 | -3.13 | 5.00E-05 | 0.01 |
| ENST00000355754 | ENSG00000162654 | GBP4 | -3.13 | 5.00E-05 | 0.01 |
| ENST00000372930 | ENSG00000126950 | TMEM35 | -3.22 | 5.00E-05 | 0.01 |
| ENST00000296575 | ENSG00000164161 | HHIP | -3.48 | 5.00E-05 | 0.01 |
| ENST00000326245 | ENSG00000179914 | ITLN1 | -3.49 | 0.0001 | 0.02 |
| ENST00000299502 | ENSG00000197632 | SERPINB2 | -3.50 | 5.00E-05 | 0.01 |
| ENST00000261292 | ENSG00000101670 | LIPG | -3.65 | 5.00E-05 | 0.01 |
| ENST00000237612 | ENSG00000118777 | ABCG2 | -3.81 | 5.00E-05 | 0.01 |
| ENST00000397463 | ENSG00000150551 | LYPD1 | -3.98 | 5.00E-05 | 0.01 |
| ENST00000381405 | ENSG00000164283 | ESM1 | -4.14 | 5.00E-05 | 0.01 |
| ENST00000295461 | ENSG00000163293 | NIPAL1 | -4.22 | 5.00E-05 | 0.01 |
| ENST00000261405 | ENSG00000110799 | VWF | -4.25 | 5.00E-05 | 0.01 |
| ENST00000378827 | ENSG00000125845 | BMP2 | -4.27 | 5.00E-05 | 0.01 |
| ENST00000263174 | ENSG00000099260 | PALMD | -4.36 | 5.00E-05 | 0.01 |
| ENST00000395201 | ENSG00000162595 | DIRAS3 | -4.39 | 5.00E-05 | 0.01 |
| ENST00000271348 | ENSG00000143140 | GJA5 | -4.86 | 5.00E-05 | 0.01 |
| ENST00000409852 | ENSG00000163273 | NPPC | -4.93 | 5.00E-05 | 0.01 |
| ENST00000282849 | ENSG00000140873 | ADAMTS18 | -6.48 | 5.00E-05 | 0.01 |
| ENST00000297316 | ENSG00000164736 | SOX17 | -6.48 | 5.00E-05 | 0.01 |
| ENST00000373674 | ENSG00000164530 | PI16 | -8.28 | 5.00E-05 | 0.01 |

### Example 4: Network and functional enrichment analyses

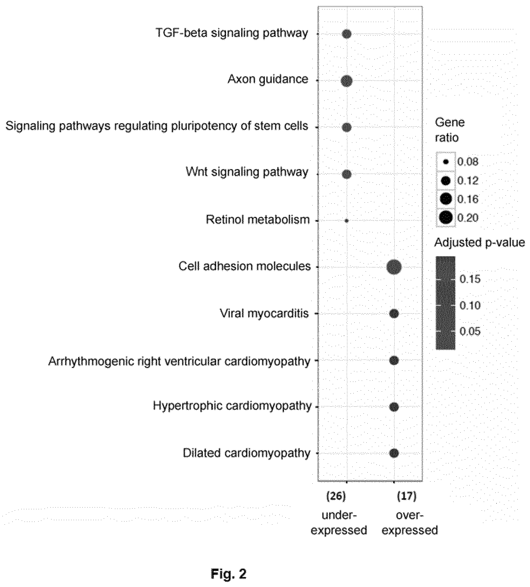

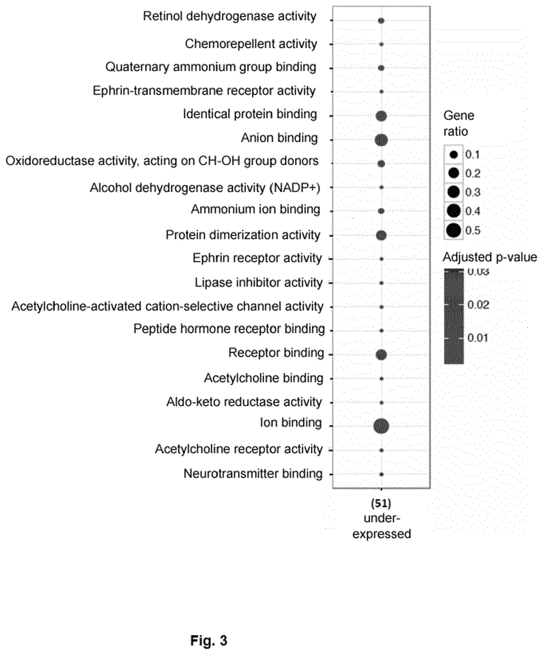

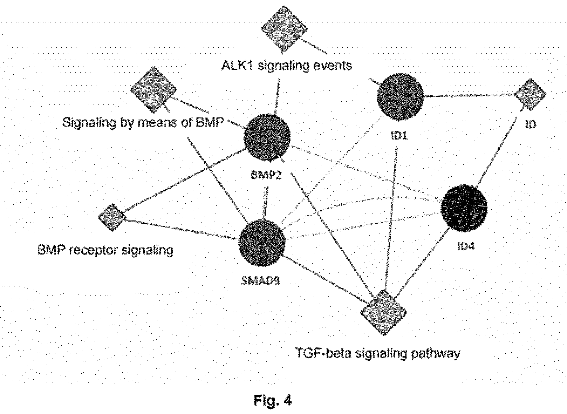

A functional and gene network analysis was then performed by means of an enrichment analysis that allows identifying combinations of significant annotations associated with the generated lists of DEG and DEI for identifying functional relationships between symptomatic and asymptomatic samples. The enrichment analysis based on bioconductors using as databases GO (Gene Ontology), KEGG (Kyoto Encyclopedia of Genes and Genome); Reactome, DO (disease ontology), OMIM (Online Mendelian Inheritance in Man), and the Cincinnati Children's Hospital Medical centre (Consortium, G. O. Nucleic Acids Res. 43, D1049-D1056 (2015); Kanehisa, M. & Goto, S. Nucleic Acids Res. 28, 27-30 (2000); Fabregat, A. et al. Nucleic Acids Res. 44, D481-D487 (2016); Yu, G.. et al. Bioinformatics 31, 608-609 (2015), generated a list of annotation groups which are significantly different between S and A. Although the analysis based on bioconductors allowed identifying functional categories common for a gene cluster, these categories between gene clusters are next compared by means of implementing the algorithm proposed by the ClusterProfiler tool. This tool, which was used to compare gene clusters (i.e., genes downregulated and upregulated in S compared to A), allowed seeing the compared biological categories between gene clusters. The functional profiles identified based on KEGG, GO, Reactome, and DO are illustrated in Figure 2, Figure 3. The comparison based on KEGG (Figure 2) identified the TGF-β signaling pathway, the axon guidance, and cell adhesion molecules (CAMs) as the categories with the highest GeneRatio number and lowest *P*_{adjust} value. In the comparison based on GO, retinol dehydrogenase activity was identified as the category with the lowest *P_{adjust}* (Figure 3). Furthermore, the network analysis of functional interactions was performed using several sources of information such as interaction of proteins, genomics, pathways, etc., using the program called GeneMANIA (Gene Multiple Association Network Integration Algorithm) (Montojo, J., et al., F1000Research 3, 2014). Figure 4 shows the functional gene network obtained by means of GeneMANIA including clusters related to TFG-β signaling, axon guidance, cell adhesion molecules, and ALK1 signaling.

An additional network analysis was then performed to identify a central network from the functional network generated with GeneMANIA with the MCODE (Molecular Complex Detection) method (Saito, R. et al. Nat Meth 9, 1069-1076 2012). MCODE performs a network clustering step, which reduces unconnected nodules in network clusters. Therefore, the final cluster identified by MCODE was the one with the highest specificity and it showed that the TGF-β, BMP, and ALK1 signaling pathways arise as a functional process, distinguishing isolated SMCs for symptomatic or asymptomatic carotid plaques. The identification in this network cluster analysis of 3 downregulated genes/isoforms *[BMP2* (DEG: FC -1.9; *P_{adjust}* 0.004 / DEI: FC-4.27; *P_{adjust}* 0.01)/ *SMAD9* (DEG: FC -1.51; *P_{adjust}* 0.05) / *ID1* (DEG: FC - 1.61; *P_{adjust}* 0.03 / DEI: FC-1.99; *P_{adjust}* 0.01)] and one upregulated gene/isoform *[ID4* (DEG: FC 155; *P_{adjust}* 0.09 / DEI: FC 1.89; *P_{adjust}* 0.01)] in SMCs associated with plaque destabilization (Figure 4) is also notable.

### Example 5: Biomarker validation

To carry out the validation, 120 genes from the list of 93 genes were selected, and furthermore, genes which showed in the RNAseq study FC values greater than 1.2 or less than -1.2 were also selected. The capacity of these genes to discriminate between symptomatic and asymptomatic patients was tested in a 190-patient cohort. To that end, the gene expression was determined from mRNA extracted from the entire plaque (in the preceding case it was in a cell type isolated from the plaque) using the Fluidigm platform (Biomark HD System) using EvaGreen technology.

The results have allowed validating 22 genes from among those that showed expression change values between 1.2 and 1.5 and -1.2 and -1.5, which are shown in bold print in Table 5.

## Claims

1. A method for diagnosing unstable atherosclerotic plaques in a subject which comprises:
(i) determining the expression level of at least one gene selected from the group of genes shown in Table 1 or from the group of genes shown in Table 2 in a biological sample from said subject, and
(ii) comparing the expression level obtained in (i) with a corresponding reference value,
wherein if the at least one gene determined in step (i) is one of genes 1-33 from Table 1 and its expression is increased with respect to the reference value, and/or wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is diagnosed with unstable atherosclerotic plaque.

2. The method according to claim 1, wherein the method comprises determining the expression level of at least one gene from group 1, at least one gene from group 2, at least one gene from group 3, at least one gene from group 4, at least one gene from group 5, at least one gene from group 6, at least one gene from Table 2, and/or at least one gene from Table 3.

3. The method according to claim 2, wherein the method comprises determining the expression level of all the genes from group 1.

4. The method according to claim 2, wherein the method comprises determining the expression level of all the genes from group 2.

5. The method according to claim 2, wherein the method comprises determining the expression level of all the genes from group 3.

6. The method according to claim 2, wherein the method comprises determining the expression level of all the genes from group 4.

7. The method according to claim 2, wherein the method comprises determining the expression level of all the genes from group 5.

8. The method according to claim 2, wherein the method comprises determining the expression level of all the genes from group 6.

9. The method according to claim 1, wherein the method comprises determining the expression level of all the genes from Table 2.

10. The method according to claim 2, wherein the method comprises determining the expression level of all the genes from Table 3.

11. The method for diagnosing unstable atherosclerotic plaques according to any of claims 2 to 8, wherein the method comprises determining the expression level of all the genes from group 1, all the genes from group 2, all the genes from group 3, all the genes from group 4, all the genes from group 5, and all the genes from group 6.

12. The method for diagnosing unstable atherosclerotic plaques according to claim 1, wherein the method comprises determining the expression level of all the genes from Table 1.

13. The method according to any of claims 1 to 12, wherein the biological sample is a sample containing cells from an atherosclerotic plaque.

14. The method according to any of claims 1 to 13, wherein the cells from an atherosclerotic plaque are smooth muscle cells.

15. The method according to claim 14, wherein the smooth muscle cells are MYH11-positive and PECAM1-negative.

16. The method according to any of claims 1 to 15, wherein the biological sample is a sample containing a mixture of cells from the entire atherosclerotic plaque.

17. The method according to any of claims 1-16, wherein the reference value of a gene from Table 1 or from Table 2 corresponds to the expression level of said gene in an atherosclerotic plaque cell sample from a subject who has stable atherosclerotic plaques.

18. The method according to any of claims 1-17, wherein the expression level is determined by means of RNA sequencing techniques.

19. A prognostic method for determining the probability of a subject suffering from a cerebrovascular disease which comprises
(i) determining the expression level of at least one gene selected from the group of genes shown in Table 1 or from the group of genes shown in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject has a high probability of suffering from a cerebrovascular disease.

20. The method according to claim 19, wherein the method comprises determining the expression level of at least one gene from group 1, at least one gene from group 2, at least one gene from group 3, at least one gene from group 4, at least one gene from group 5, at least one gene from group 6, at least one gene from Table 2, and/or at least one gene from Table 3.

21. The method according to claim 20, wherein the method comprises determining the expression level of all the genes from group 1.

22. The method according to claim 20, wherein the method comprises determining the expression level of all the genes from group 2.

23. The method according to claim 21, wherein the method comprises determining the expression level of all the genes from group 3.

24. The method according to claim 21, wherein the method comprises determining the expression level of all the genes from group 4.

25. The method according to claim 21, wherein the method comprises determining the expression level of all the genes from group 5.

26. The method according to claim 21, wherein the method comprises determining the expression level of all the genes from group 6.

27. The method according to claim 19, wherein the method comprises determining the expression level of all the genes from Table 2.

28. The method according to claim 21, wherein the method comprises determining the expression level of all the genes from Table 3.

29. The method according to any of claims 19 to 28, wherein the method comprises determining the expression level of all the genes from group 1, all the genes from group 2, all the genes from group 3, all the genes from group 4, all the genes from group 5, and all the genes from group 6.

30. The prognostic method according to claim 29, wherein the method comprises determining the expression level of all the genes from Table 1.

31. The method according to any of claims 18 to 30, wherein the biological sample is a sample containing cells from an atherosclerotic plaque.

32. The method according to claim 31, wherein the cells from an atherosclerotic plaque are smooth muscle cells.

33. The method according to claim 32, wherein the smooth muscle cells are MYH11-positive and PECAM1-negative.

34. The method according to any of claims 19 to 30, wherein the biological sample is a sample containing a mixture of cells from the entire atherosclerotic plaque.

35. The method according to any of claims 19 to 34, wherein the reference value corresponds to the expression level of said gene in an atherosclerotic plaque cell sample from a subject who has not suffered from a cerebrovascular disease.

36. The method according to any of claims 19 to 35, wherein the expression level is determined by means of RNA sequencing techniques.

37. The method according to any of claims 19 to 36, wherein the cerebrovascular disease is apoplexy.

38. A method for selecting a patient susceptible to being treated with a therapy for removing or stabilizing the carotid atherosclerotic plaque which comprises:
(i) determining the expression level of at least one gene selected from the group of genes shown in Table 1 or from the group of genes shown in Table 2 in a biological sample from said subject, and
(ii) comparing the expression level obtained in (i) with a reference value,
wherein if the at least one gene determined in step (i) is one of genes 1-33 from Table 1 and its expression is increased with respect to the reference value, and/or
wherein if the at least one gene determined in step (i) is one of genes 1-6 from Table 2 and its expression is increased with respect to the reference value, and/or
wherein if the at least one gene determined in step (i) is one of genes 34-93 from Table 1 and its expression is reduced with respect to the reference value, and/or
wherein if the at least one gene determined in step (i) is one of genes 7-11 from Table 2 and its expression is reduced with respect to the reference value,
then the subject is susceptible to being treated for removing or stabilizing the carotid atherosclerotic plaque.

39. The method according to claim 38, wherein the therapy for removing the carotid atherosclerotic plaque is carotid endarterectomy or the therapy for stabilizing the plaque is statins or antiaggregants.

40. The method according to any of claims 38 or 39, wherein the method comprises determining the expression level of at least one gene from group 1, at least one gene from group 2, at least one gene from group 3, at least one gene from group 4, at least one gene from group 5, at least one gene from group 6, at least one gene from Table 2, and/or at least one gene from Table 3.

41. The method according to claim 40, wherein the method comprises determining the expression level of all the genes from group 1.

42. The method according to claim 40, wherein the method comprises determining the expression level of all the genes from group 2.

43. The method according to claim 40, wherein the method comprises determining the expression level of all the genes from group 3.

44. The method according to claim 40, wherein the method comprises determining the expression level of all the genes from group 4.

45. The method according to claim 40, wherein the method comprises determining the expression level of all the genes from group 5.

46. The method according to claim 40, wherein the method comprises determining the expression level of all the genes from group 6.

47. The method according to claim 38, wherein the method comprises determining the expression level of all the genes from Table 2.

48. The method according to claim 40, wherein the method comprises determining the expression level of all the genes from Table 3.

49. The method for diagnosing unstable atherosclerotic plaques according to any of claims 38 to 48, wherein the method comprises determining the expression level of all the genes from group 1, all the genes from group 2, all the genes from group 3, all the genes from group 4, all the genes from group 5, and all the genes from group 6.

50. The method according to any of claims 37 or 38, wherein the method comprises determining the expression level of all the genes from Table 1.

51. The method according to any of claims 38 to 50, wherein the biological sample is a sample containing cells from an atherosclerotic plaque.

52. The method according to claim 51, wherein the cells from an atherosclerotic plaque are smooth muscle cells.

53. The method according to claim 52, wherein the smooth muscle cells are MYH11-positive and PECAM1-negative.

54. The method according to any of claims 38 to 50, wherein the biological sample is a sample containing a mixture of cells from the entire atherosclerotic plaque.

55. The method according to any of claims 38 to 54, wherein the reference value of a gene from Table 1 or from Table 2 corresponds to the expression level of said gene in an atherosclerotic plaque cell sample from a subject who has stable atherosclerotic plaques or who has responded to said treatment.

56. The method according to any of claims 38 to 55, wherein the expression level is determined by means of RNA sequencing techniques.

57. A kit comprising reagents suitable for determining the expression levels of at least one gene selected from the group of genes shown in Table 1 or from the group of genes shown in Table 2, and optionally reagents for determining the expression levels of one or more housekeeping genes.

58. The kit according to claim 57, wherein said kit comprises reagents suitable for determining the expression level of at least one gene from group 1, at least one gene from group 2, at least one gene from group 3, at least one gene from group 4, at least one gene from group 5, at least one gene from group 6, at least one gene from Table 2, and/or at least one gene from Table 3.

59. The kit according to claim 58, wherein said kit comprises reagents suitable for determining the expression level of all the genes from group 1.

60. The kit according to claim 58, wherein said kit comprises reagents suitable for determining the expression level of all the genes from group 2.

61. The kit according to claim 58, wherein said kit comprises reagents suitable for determining the expression level of all the genes from group 3.

62. The kit according to claim 58, wherein said kit comprises reagents suitable for determining the expression level of all the genes from group 4.

63. The kit according to claim 58, wherein said kit comprises reagents suitable for determining the expression level of all the genes from group 5.

64. The kit according to claim 58, wherein said kit comprises reagents suitable for determining the expression level of all the genes from group 6.

65. The kit according to claim 58, wherein said kit comprises reagents suitable for determining the expression level of all the genes from Table 2.

66. The kit according to claim 58, wherein said kit comprises reagents suitable for determining the expression level of all the genes from Table 3.

67. The kit according to any of claims 58 or 59, wherein said kit comprises reagents suitable for determining the expression level of all the genes from group 1, all the genes from group 2, all the genes from group 3, all the genes from group 4, all the genes from group 5, and all the genes from group 6.

68. The kit according to claim 67, wherein said kit comprises reagents suitable for determining the expression level of all the genes from Table 1.

69. Use of a kit according to any of claims 57 to 68 for diagnosing unstable atherosclerotic plaques in a subject, for determining the probability of a subject suffering from a cerebrovascular disease, or for selecting a patient susceptible to being treated with a therapy for removing or stabilizing the carotid atherosclerotic plaque.
